# EUROPEAN PATENT SPECIFICATION

(11) **EP 4 115 849 B1**
(45) Date of publication and mention of the grant of the patent: **30.09.2026**
(21) Application number: 21305950.4
(22) Date of filing: 09.07.2021
(51) Int. Cl.: A61F 2/00, A61L 31/14, A61L 31/06, A61L 31/04

(54) **COMPOSITE IMPLANT INCUDING A MESH AND A FOAM AND METHODS ASSOCIATED THEREWITH**
ZUSAMMENGESETZTES IMPLANTAT MIT EINEM NETZ UND EINEM SCHAUMSTOFF UND DAMIT VERBUNDENE VERFAHREN
IMPLANT COMPOSITE COMPRENANT UNE MAILLE ET UNE MOUSSE ET PROCÉDÉS ASSOCIÉS

(43) Date of publication of application: 11.01.2023
(73) Proprietor: Sofradim Production, 01600 Trevoux (FR)
(72) Inventor: SERENI, Nicolas, 69100 Villeurbanne (FR); BRUNE, Thierry, 69640 Jarnioux (FR)
(74) Representative: Germain Maureau

(56) References cited:
- WO-A1-2011/027087
- US-A1- 2013 256 375
- US-A1- 2018 325 518

## Description

### BACKGROUND

### Technical Field:

The present disclosure relates generally to composite implants including a mesh and a foam, and in particular composite implants including a mesh including at least one grip member and a foam configured to be compressed thereon. The composite implants are suitable for the treatment or repair of soft tissue openings or wounds.

### Related Art:

A self-fixating mesh including grips to ensure fixation on biological tissues without using additional fixation was developed initially for open surgery. Later, with an increase of laparoscopic surgeries, the self-fixating mesh was adapted for the needs of this surgical approach. To introduce the self-fixating mesh through trocars commonly used in laparoscopic surgery, the self-fixating mesh may need to be rolled on itself. When rolled, the grips on the mesh may become entangled in the body of any overlapping portion of the mesh, which can interfere with the subsequent deployment of the mesh at the implantation site.

WO2011027087 attempts to limit the self-gripping effect by proposing to cover the individual grips with a film coating made of a water-soluble solution. A method is described which coats the grips with a polyethylene glycol (PEG) solution using a brush. After drying of the polyethylene glycol solution, the film coated grips have the capacity to slide over one another to limit gripping on the back textile when rolled. When the coated grips are brought in contact with biological tissues at the implantation site, the film coating covering the grips solubilizes little by little and thus detaches from the individual grips over a period of time. Authors describe in addition that the necessary solubilization time allows the surgeon to position (and reposition) the mesh with respect to the surrounding tissues. However, the method of coating the individual grips via brushing does not provide the ability to well control the homogeneity of the coating over each of the individual grips. Thus, due to any possible heterogeneities linked to the film coating, the dissolution time of the coating, and ultimately the self-fixation performance of the grips, will not be the same across all the grips and/or will not be determinable by the surgeon until after implantation.

In addition, because the grips do not begin to attach to the tissue until the coating is dissolves, and the film coating does not start to dissolve until implanted and placed into contact with the tissue, the surgeon is prevented from selecting the order in which portions of the mesh attach to tissue. Rather, this decision is effectively predetermined during the manufacturing process of the mesh. There is also a lack of control to fix and/or refix the mesh at the right position when desired.

WO2011/026987 describes another solution to limit the self-gripping effect by covering the backside of the mesh (opposite the side of the mesh with the grips) with a continuous bioabsorbable layer. The bioabsorbable layer is described as an impenetrable barrier designed to limit the self-gripping when the mesh is rolled on itself. The grips are not allowed to penetrate in the body (backside of the mesh) of the mesh because of the bioabsorbable layer. Furthermore, this layer is resorbed from a few hours to a few months under physiological conditions, thus acting as a barrier to post-operative tissue adhesions. However, to whatever extent the continuous layer prevents the self-gripping to the mesh itself when rolled, the continuous layer does not affect the gripping to surrounding tissues during mesh insertion because the bioabsorbable layer is not positioned on the grips. Also, due to the lengthy resorption rate, the continuous layer would not be effective if applied directly to the grips because the surgical procedure requires the mesh to attach to tissue, after proper orientation, before the surgical procedure is finalized, and not weeks to months later.

U.S. Patent No. 9629703 proposes to cover the grips with a dissolvable matrix layer. Authors, without giving an example of the corresponding technology, describe the possibility to cover the gripping side of a self-fixating mesh to increase the time before the grips fix to the biological tissues. The dissolvable matrix may improve the handling of the self-fixating mesh (insertion, deployment) and may offer the possibility to the surgeon to (re)position the mesh at the right location. After positioning of the mesh properly, the mesh does not attach to the tissue until the layer dissolves and thus the mesh must be maintained in contact with the tissues until at least a sufficient portion of the layer dissolves. To help with the dissolution of the matrix material, authors propose adding a liquid solvent such as saline to the matrix. Moreover, authors discuss the possibility to have a matrix layer, which is dissolved in a controlled manner (matrix layer formulation or/and add a liquid solvent). However, it is difficult if not impossible to predict the amount of natural body fluid provided by the surgery site for each patient, and to predict convective movements of the fluid provided by the mesh insertion, deployment and (re)positioning for each practical surgery. Also, adding a layer during the manufacturing of the implant, just to increase the time before the grips are able to self-grip and/or grip to the tissues, may not be enough and does not offer the possibility for the surgeon to determine which locations of the mesh to affix first, post-manufacturing.

US2013/0256375A1 discloses a tissue stapler having a thickness compensator. US2018/0325518A1 discloses a recessed surgical fastening device. Thus, currently, there are several technologies, which attempt to limit the self-gripping effect when rolled and the premature gripping to surrounding tissues upon insertion of self-fixating mesh. All these technologies are based on the use of a dissolvable layer to temporally inhibit gripping capacity of the grips. However, due to the coating method and/or the structure of the matrix layer, all these technologies have limitations and do not offer a complete control to the surgeons for fixing the mesh at the right place, and at the specific time desired.

### SUMMARY

The present disclosure describes composite implants suitable for soft tissue repair, the composite implants include at least one mesh having grip members extending therefrom and a compressible foam positioned on at least a portion of the grip members. The composite implants described herein are configured to provide the medical staff or surgeon the ability to determine which portions or locations of the mesh or implant to activate the gripping ability of the grip members thereon, and/or those grip members not to activate, post-manufacturing of the implant. In addition, the composite implants described herein may be configured to determine the activation or inactivation of grip members of the composite implant, regardless or independent of the dissolution rate of the compressible foam.

A composite implant is provided according to claim 1.

In some embodiments, the compressible foam in the first configuration has a first thickness which covers the entire body of each of the plurality of grip members. The first thickness of the foam being greater than or equal to a length of a body of each of the plurality of grip members.

In some embodiments, the compressible foam in the first configuration covers only a portion of the body of each of the plurality of grip members creating a gap between the first face of the mesh and a second surface of the compressible foam in the first configuration. The first thickness of the foam being smaller than a length of a body of each of the plurality of grip members. The gap remaining even after the foam transitions to the second configuration.

The present disclosure further describes an example of a composite implant including a mesh, a flap mesh, and a compressible foam. The mesh includes a first mesh face, a second mesh face opposite the first mesh face, and a slit defined therethrough. The flap mesh including a first flap face and a second flap face opposite the first flap face, the flap mesh extending across the slit and including a first portion of the second flap face overlapping a first portion of the first face of the mesh. A plurality of grip members extending from one of the first portion of the first face of the mesh or the first portion of the second flap face, each of the plurality of grip members including a body extending between a first attached end and a second free end. A compressible foam covering at least the second free ends of the plurality of grip members, the compressible foam configured to transition from a first foam configuration having a first thickness to a second foam configuration having a second thickness smaller than the first thickness. In some embodiments, the foam in the second configuration either directly or indirectly exposes the second ends of the plurality of grip members.

Methods of soft tissue repair are also described. In some examples, a method of soft tissue repair includes: inserting a composite implant into a site of implantation of a patient near a wound in the soft tissue in need of repair, the composite implant including a mesh having a first face and a second face opposite the first face, a plurality of grip members extending from the first face of the mesh, each of the plurality of grip members including a body extending between a first end attached to the mesh and a second end free of the mesh, and a compressible foam covering at least the second ends of the plurality of grip members, the compressible foam configured to transition from a first foam configuration having a first thickness to a second foam configuration having a second thickness smaller than the first thickness, and the grip members are configured to transition from a first inactive gripping configuration to a second active gripping configuration upon removal of the foam; deploying the composite implant across the wound in the soft tissue; applying pressure to a portion of the compressible foam to transition from the first foam configuration to the second foam configuration having a second thickness less than the first thickness thereby; and transitioning the grip members from the inactive gripping configuration to the active gripping configuration to fixate the grip members and/or implant to the tissue. In some embodiments, the foam in the second configuration either directly or indirectly exposes the second ends of the plurality of grip members.

A method of manufacturing a composite implant is provided according to claim 8.

In some embodiments, the biocompatible polymeric foaming agent is hydroxypropyl methylcellulose and the biocompatible plasticizer is glycerin and/or sorbitol.

### BRIEF DESCRIPTION OF THE DRAWINGS

The accompanying drawings, which are incorporated in and constitute a part of the specification, illustrate embodiments of the disclosure and, together with a general description of the disclosure given above, and the detailed description of the embodiments given below, serve to explain the principles of the disclosure.
FIG. 1 is a schematic cross-sectional view of an implantable mesh as described in at least one embodiment herein;
FIGS. 2A and 2B are schematic cross-sectional views of at least one grip member as described in at least one embodiment herein;
FIGS. 3A-3D are schematic cross-sectional views of at least one composite implant as described in at least one embodiment herein;
FIGS. 4A-4D are schematic cross-sectional views of at least one composite implant as described in at least one embodiment herein;
FIGS. 5A-5B are schematic cross-sectional views of at least one composite implant as described in at least one embodiment herein;
FIGS. 6A-6B are schematic cross-sectional views of at least one composite implant as described in at least one embodiment herein;
FIG. 7 is a schematic cross-sectional views of at least one composite implant as described in at least one embodiment herein;
FIGS. 8A-8D are schematic top views of at least one composite implant as described in at least one embodiment herein;
FIGS. 9A-9E are schematic top views of at least one composite implant as described in at least one embodiment herein;
FIG. 10 is top view of a wet non-flow foam material combined with an implantable mesh as described in at least one embodiment herein;
FIG. 11A is top view of a wet non-flow foam material combined with a mold or frame as described in at least one embodiment herein;
FIG. 11B is top view of a wet non-flow foam layer within a mold or frame as described in at least one embodiment herein;
FIG. 11C is a schematic view of a method of indirectly applying a wet non-flow material to an implantable mesh to form various composite implants as described in at least one embodiment herein;
FIG. 12A is an image of a scanning electron microscope of an implantable mesh as described in at least one embodiment herein;
FIGS. 12B and 12C are images of a scanning electron microscope of various composite implants as described in at least one embodiment herein;
FIGS. 13A-13C are perspective views of folding or rolling a self-fixating mesh free of a compressible foam as described in at least one embodiment herein;
FIGS. 13D-13F are perspective views of folding or rolling a composite implant as described in at least one embodiment herein;
FIG. 14A is a perspective of an incision in a tissue as described in at least one embodiment herein;
FIGS. 14B-14D are perspective views of inserting and folding or rolling of a self-fixating mesh free of a compressible foam as described in at least one embodiment herein;
FIGS. 14E-14G are perspective views of inserting and folding or rolling of incomposite implant as described in at least one embodiment herein;
FIGS. 15A-15E are perspective views of applying pressure indirectly to a composite implant and attaching the composite implant to tissue as described in at least one embodiment herein;
FIG. 16 is a chart which compares the gripping performance of the self-fixating mesh with the gripping performance of the composite implants as described herein after removal and/or dissolution of the foam from at least the grip members;
FIGS. 17A and 17B are images of a scanning electron microscope of a compressible foam as described in at least one embodiment herein;

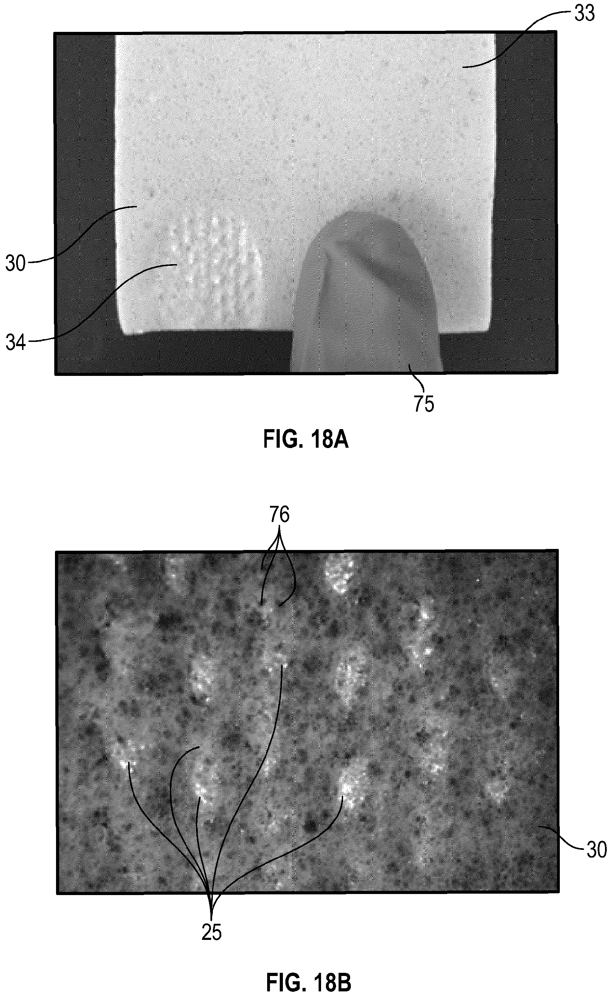

FIG. 18A is a top view of applying pressure directly to the compressible foam of a composite implant and indirectly exposing the free ends of the grip members of the composite implant as described in at least one embodiment herein;
FIG. 18B is an image of a scanning electron microscope of a compressible foam in a second configuration and indirectly exposing the free ends of the grip members of the composite implant as described in at least one embodiment herein;
FIGS. 19A and 19B are charts which compare the gripping force needed to remove a self-fixating mesh free of a compressible foam and various composite implants from tissue as described in at least one embodiment herein;
FIG. 20 is a chart displaying the results of inactivation and activation gripping tests as described in at least one embodiment herein; and
FIGS. 21A-21D are images of an abrasion tester used in methods of assessing the compressible foams described herein.

### DETAILED DESCRIPTION

The present disclosure relates to composite implants including an implantable mesh and a compressible foam. The implantable mesh is configured to grip to tissue and/or other portions of the implantable mesh. The compressible foam is configured to transition from a first configuration having a first initial thickness to a second configuration having a second subsequent thickness.

### Implantable Mesh

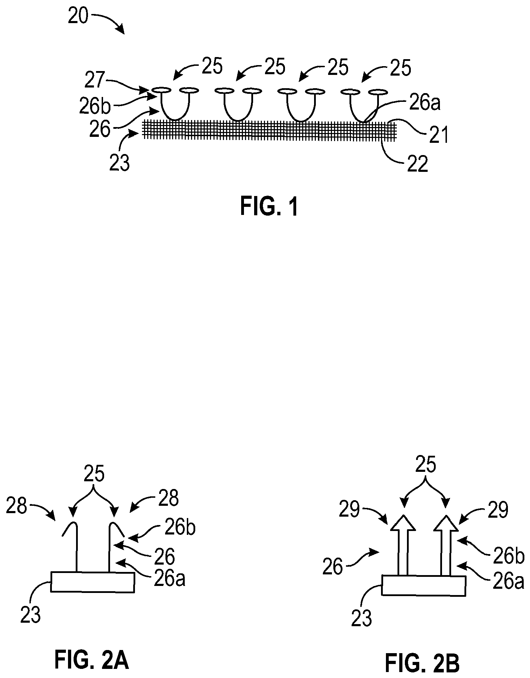

FIG. 1 depicts a schematic cross-sectional view of an implantable mesh 20 as described herein. The implantable mesh 20 includes a mesh body 23 having a first face 21 and a second face 22 opposite the first face 21, the first face 21 including one or more grip members 25. Each grip member 25 includes a first end portion 26a of a grip body 26 attached to or extending from the mesh body 23 (and/or one of the mesh faces 21, 22). A second free end portion 26b, opposite the first end portion 26a, extends farthest away from the mesh body 23 (and/or one of the mesh faces 21, 22) and may include a head portion 27 configured to grip to tissue and/or grip to portions of the mesh 20 when overlapped, i.e., folded, rolled, etc.

The mesh 20 of FIG. 1 is one non-limiting example of a self-fixating mesh. The mesh 20 of FIG. 1 does not include a compressible foam located on any portion thereof, particularly the grip members 25, and as such is not yet a composite implant as described herein.

The implantable mesh, including the mesh body and optionally the one or more grip members, can be formed from one or more intertwined filaments. The filaments may be monofilaments or multi-filaments. The implantable mesh, and particularly the mesh body, may be formed using any suitable method known to those of ordinary skill including, but not limited to, weaving, knitting, braiding, crocheting, embroidering, and the like.

In some embodiments, the implantable mesh includes a knit mesh body. In some embodiments, the implantable knit mesh may be knitted on a warp knitting machine, of the tricot or Raschel type, with at least two or three guide bars. In some embodiments, the implantable mesh may be made as provided in any of the U.S. Patent Nos. 6,596,002; 7,331,199; 9,750,595; 9,186,235; 9,510,927.

In some embodiments, the implantable mesh includes a two-dimensional mesh body. For example, the mesh body may include one or more filaments intertwined in a generally planar or horizontal crisscrossing pattern, i.e., weft/warp pattern, forming a mesh body including a single layer of intertwined filament(s).

In some embodiments, the implantable mesh includes a three-dimensional mesh body. For example, the mesh body may include one or more filaments intertwined in both a horizontal and vertical pattern forming a mesh body including two or more layers of intertwined filament(s) often connected to each other by a spacer filament, i.e., a filament that extends between and connects the two or more layers, defining a space or depth between the two or more layers.

The implantable mesh, and particularly the filaments that form the mesh, can be made from any biocompatible material, including bioabsorbable materials, non-bioabsorbable materials, and combinations thereof. The term "bioabsorbable" as used herein is defined to include both biodegradable and bioabsorbable materials. By bioabsorbable, it is meant that the materials decompose, or lose structural integrity under body conditions (e.g. enzymatic degradation or hydrolysis) or are broken down (physically or chemically) under physiologic conditions in the body such that the degradation products are excretable or absorbable by the body.

Representative natural bioabsorbable materials include, but are not limited to: polysaccharides, such as alginate, dextran, chitin, hyaluronic acid, cellulose, collagen, gelatin, fucans, glycosaminoglycans, and chemical derivatives thereof (substitutions and/or additions of chemical groups, for example, alkyl, alkylene, hydroxylations, oxidations, and other modifications routinely made by those skilled in the art); and proteins, such as albumin, casein, zein, silk, and copolymers and blends thereof, alone or in combination with synthetic polymers.

Synthetically modified natural polymers include, but are not limited to, cellulose derivatives, such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt.

Representative synthetic bioabsorbable polymers include, but are not limited to, polyhydroxy acids prepared from lactone monomers, such as glycolide, lactide, caprolactone, ε-caprolactone, valerolactone, and δ-valerolactone, as well as pluronics, carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s; polyalkylene oxalates; polyoxaesters; polyanhydrides; polyortho esters; and copolymers, block copolymers, homopolymers, blends, and combinations thereof.

Some non-limiting examples of suitable non-bioabsorbable materials include polyolefins, such as polyethylene and polypropylene including atactic, isotactic, syndiotactic, and blends thereof; polyethylene glycols; polyethylene oxides; ultra-high molecular weight polyethylene; copolymers of polyethylene and polypropylene; polyisobutylene and ethylene-alpha olefin copolymers; fluorinated polyolefins, such as fluoroethylenes, including expanded polytetrafluoroethylene (ePTFE) and condensed polytetrafluoroethylene c(PTFE), fluoropropylenes, fluoroPEGSs, and polytetrafluoroethylene; polyamides, such as nylon and polycaprolactam; polyamines; polyimines; polyesters, such as polyethylene terephthalate and polybutylene terephthalate; aliphatic polyesters; polyethers; polyether-esters, such as polybutester; polytetramethylene ether glycol; 1,4-butanediol; polyurethanes; acrylic polymers and copolymers; modacrylics; vinyl halide polymers and copolymers, such as polyvinyl chloride; polyvinyl alcohols; polyvinyl ethers, such as polyvinyl methyl ether; polyvinylidene halides, such as polyvinylidene fluoride and polyvinylidene chloride; polyacrylonitrile; polyaryletherketones; polyvinyl ketones; polyvinyl aromatics, such as polystyrene; polyvinyl esters, such as polyvinyl acetate; copolymers of vinyl monomers with each other and olefins, such as etheylene-methyl methacrylate copolymers, acrylonitrile-styrene copolymers, ABS resins, and ethylene-vinyl acetate copolymers; alkyd resins; polycarbonates; polyoxymethylenes; polyphosphazine; polyimides; epoxy resins; aramids, rayon; rayon-triacetate; spandex; silicones; and combinations thereof.

In some embodiments, the implantable mesh is formed from a combination of bioabsorbable and non-bioabsorbable filaments. For example, a first set of non-bioabsorbable filaments may be used to form the mesh body and a second set of absorbable filaments may be used to form the grip members extending therefrom. Other various combination of absorbable and/or non-absorbable filaments may be used to form the mesh described herein.

In some embodiments, the implantable mesh is a self-fixating mesh such as Parietex Progrip^{™} or Parietene Progrip^{™} (both commercially available from Covidien). Parietex Progrip^{™} includes a mesh body made from one or more polyester filaments and grip members extending therefrom made from a bioabsorbable material such as poly lactic acid (PLA).

In addition to the mesh body, the implantable mesh described herein include one or more grip members extending from at least some portion of, if not all, of the first and/or second mesh faces. The grip-members may take any suitable form configured to attach directly to tissue and/or other portions of the implantable mesh (or a second implantable mesh). Some non-limiting examples include spiked naps, hooks, barbs, and combinations thereof.

As shown in FIG. 1, in some embodiments, the one or more grip members 25 are spiked naps, wherein the head portion 27 is wider than the elongate body 26. Each spiked nap has a body 26 protruding from the first face 21 of the mesh body 23 and, at the second free end 26b of the body 26, a head 27 including a greater width than that of the body 26. The head 27 may be a generally spheroidal shape or a mushroom shape. The head portion 27 may include a smooth outer surface or may include a rough outer surface having asperities. The first end 26a of the body 26 of the spiked nap being interwoven with the filaments of the mesh body 23 of the implantable mesh 20.

FIGS. 2A and 2B depict the one or more grip members 25 in other possible non-limiting forms. As shown in FIG. 2A, in some embodiments, the one or more grip members 25 may include a hook design. Each grip member 25 includes an elongate body 26 protruding from the mesh body 23 and, at the second free end 26b of the body 26, the head portion 27 includes a hook. The first end 26a of the body 26 being interwoven with the filaments of the mesh body 23 of the implantable mesh 20.

As shown in FIG. 2B, in some embodiments, the one or more grip members 25 may include a barb or piercing tip design. Each grip member 25 includes an elongate body 26 protruding from the mesh body 23 and, at the second free end 26b of the body 26, the head portion 27 includes a barb or piercing tip. The first end 26a of the body 26 being interwoven with the filaments of the mesh body 23 of the implantable mesh 20.

Although the grip members are schematically depicted as spike naps throughout the remaining Figures, the mesh and/or implants described herein may include any suitable grip member configured to attach or grip to tissue including but not limited to the hook design, barb, or piercing tip designs also depicted herein, alone or in any combination.

The grip members, regardless of form, may be positioned on and/or extend from any portion of at least one face of an implantable mesh. In some embodiments, the grip members, like the mesh body, may be formed from one or more filaments which may be made from any of the bioabsorbable and/or non-bioabsorbable materials described hereinabove. In some embodiments, the grip members may be made from a bioabsorbable material, i.e., polylactic acid (PLA) or copolymers of PLA and trimethylene carbonate (TMC).

The length of each of the grip members, measured from the face of the mesh, from which the grip members project from, to the end of the second free end of the grip member, is smaller than the thickness of the implantable mesh body in which the grip members may penetrate and become entangled thereto. In some embodiments, the length of each of the grip members can be between about 0.1 and 5 millimeters. In some embodiments, the length of each of the grip members can be between about 0.5 and 3.5 millimeters. In some embodiments, the length of each of the grip members can be between about 1 and 3 millimeters. In some embodiments, the length of each of the grip members can be between about 1.5 and 2.5 millimeters.

In addition to the one or more grip members, the implantable mesh may further include a generally central opening and/or a slit. The opening being defined through the body of the mesh and configured to allow the passage of a body part therethrough, such as the spermatic chord or a portion of the bowel. The slit being a cut extending inwardly from an outer edge of the mesh. The slit separating a first portion of the mesh body from a second portion of the mesh body.

In some embodiments, the implantable mesh includes both a generally central opening and a slit extending therefrom. In such embodiments, the implantable mesh may further include a second mesh in the form a flap mesh. The flap mesh being secured to the first portion of the implantable mesh body and being configured to selectively extend across the slit to the second portion of the mesh body. In such embodiments, the one or more grip members may be positioned on the implantable mesh, the flap mesh, or both.

### Compressible Foam

The composite implants described herein also include a compressible foam. By "compressible," the foam, in a dried form and upon an application of pressure thereto, is configured to transition from a first foam configuration having a first or initial thickness to a second foam configuration having a second or subsequent thickness, wherein the second or subsequent thickness (or configuration) is smaller or thinner than the first or initial thickness (or configuration) and the second or subsequent thickness (or configuration) is maintained after the application of pressure is removed. The second or subsequent thickness (or configuration) may also be maintained prior to initial absorption or dissolution of the compressible foam in vivo.

The compressible foam defines generally a length, a width and a thickness. The length extending in a longitudinal direction, the width extending in a transverse direction, and the thickness referring to the height or verticality of the foam in a direction generally perpendicular to both the length and width. For example, the first and second thickness may be defined between a first outer surface of the foam and a second inner surface of the foam opposite the first outer surface. The inner and outer surfaces of the foam being generally parallel to the first and/or second face of the mesh body upon which the foam is generally positioned. In some embodiments, the thickness may be determined by the maximum distance between the first and second outer surfaces of the foam or first and second faces of the mesh.

The compressible foam, in both the first and second configurations, is a porous structure including open and interconnected pores. By "open and interconnected pores," the foam has an open porosity that can be accessed from outside of the foam, and that the pores communicate with one another to form a three-dimensional network throughout the foam.

The compressible foam, in the first or initial configuration (or thickness), is positioned on at least the free end, and particularly the head portion, of the one or more grip members (e.g., spiked nap, hook, barb, etc.) extending or protruding from at least one face of the mesh. The foam may extend along any length of a body of the one or more grip members. In the first or initial configuration, the second free end, and particularly the head portion, of the one or more grip members are prevented from attaching directly to tissue or other portions of the mesh (or a second mesh or mesh flap) by the compressible foam.

In some embodiments, the compressible foam, in the first or initial configuration (or thickness), may be positioned on an entire length of a body of the one or more grip members. In such embodiments, the second inner surface of the foam at least abuts up against the first or second face of the mesh upon which the compressible foam is generally positioned. Thus, no gap exists between the second inner surface of the compressible foam and the first or second face of the mesh upon which the foam is generally positioned.

In some embodiments, the compressible foam, in the first or initial configuration (or thickness), may be positioned on only a portion of the length of the body of the one or more grip members creating a gap between the first or second face of the mesh (from which the grip body extends or protrudes) and the second inner surface of the compressible foam. In such embodiments, the second inner surface of the foam does not abut up against the first or second face of the mesh.

In some embodiments, the first or initial thickness of the compressible foam (in the first configuration) may be greater than or equal to: 1mm; 1.5mm; or 2mm. In some embodiments, the first or initial thickness of the compressible foam (in the first configuration) may be less than or equal to: 1mm; 1.5mm; or 2mm. In some embodiments, the first or initial thickness of the compressible foam (in the first configuration) may range from: 0.1-3mm; 0.1-2mm; 0.25-1.5mm; 0.5-1.25mm; 1-3mm; 1.25-2.75mm; or 1.5-2.5mm. In some embodiments, the first or initial thickness of the compressible foam (in the first configuration) may be greater than or equal to a length of each of the plurality of grip members. In some embodiments, the first or initial thickness of the compressible foam (in the first configuration) may be less than or equal to a length of each of the plurality of grip members.

The compressible foam, in the second or subsequent configuration (or thickness), is positioned on at least some portion of a body of the one or more grip members (e.g., spiked nap, hook, barb, etc.). In some embodiments, the compressible foam in the second foam configuration may still cover the second free end, and particularly the head portion, of the one or more grip members thereby preventing the grip member from attaching to tissue or mesh in an inactive gripping configuration. In some embodiments, the covered grip members may be indirectly exposed via the compressed foam. In some embodiments, the covered grip members may remain hidden or concealed from sight by the compressed foam. In some embodiments, the compressible foam in the second foam configuration may not be on the second free end, and particularly the head portion, of the one or more grip members thereby directly exposing the grip member to attach to tissue in an active gripping configuration. In the active gripping configuration, the second free end, and particularly the head portion, of the one or more grip members are free of the foam and are configured to attach directly to tissue or other portions of the mesh (or a second mesh or mesh flap).

In some embodiments, the second or subsequent thickness of the compressible foam (in the second configuration) may be greater than or equal to: 1mm; 1.5mm; or 2mm. In some embodiments, the second or subsequent thickness of the compressible foam (in the second configuration) may be less than or equal to: 1mm; 1.5mm; or 2mm. In some embodiments, the second or subsequent thickness of the compressible foam (in the second configuration) may range from: 0.1-3mm; 0.1-2mm; 0.25-1.5mm; 0.5-1.25mm; 1-3mm; 1.25-2.75mm; or 1.5-2.5mm.

In some embodiments, the first or initial thickness of the compressible foam (in the first configuration) may be greater than or equal to: 1mm; 1.5mm; or 2mm, and the second or subsequent thickness of the compressible foam (in the second configuration) may be less than or equal to: 1mm; 1.5mm; or 2mm, respectively. In some embodiments, the first or initial thickness of the compressible foam (in the first configuration) may be greater than: 1mm; 1.5mm; or 2mm, and the second or subsequent thickness of the compressible foam (in the second configuration) may be less than or equal to: 1mm; 1.5mm; or 2mm, respectively, wherein the second or subsequent thickness is less than the first or initial thickness.

In some embodiments, the first or initial thickness of the compressible foam (in the first configuration) may range from: 1-4mm; 1.25-3.75mm; 1.5-3.5mm; 1.75mm to 3.25mm; or 2-3mm, and the second or subsequent thickness of the compressible foam (in the second configuration) may range from: 0.01-1mm; 0.1-1.25mm; 0.25-1.5mm; 0.5-1.75mm; or 0.75-2mm, respectively, wherein the second or subsequent thickness is less than the first or initial thickness.

In some embodiments, the compressible foam may be positioned on the second free end, and particularly the head portion, of every grip member spread across at least one face of the mesh. In some embodiments, the compressible foam may be positioned on the second free end, and particularly the head portion, of only select grip members spread across at least one face of the mesh forming a compressible foam pattern. The pattern of the compressible foam may take any shape, including but not limited to, stripes, polygons, circles, ovals, rings, dots, dashes, arrows, numbers, letters, tear drops, etc.

The compressible foams described herein may be constituted of at least one biocompatible polymeric foaming agent, and optionally at least one biocompatible plasticizer, at least one biocompatible additive, or both. Each of the foaming agent, the plasticizer, and the additive being soluble within physiological conditions at the site of the soft tissue repair, i.e., the site of a hernia, prolapse, or stoma, or similar fluids such as saline. By "soluble within physiological conditions of the site," the selected material is soluble according to the pH, temperature and/or osmolarity of the tissues on the hernia site. For example, in some embodiments, the dry foam may include gelatins that are soluble at 37°C but not at 20°C, and/or the dry foam may include sodium hyaluronate soluble at a pH=7 but not at a pH=3, and/or the dry foam may include sodium alginates soluble into a solution containing sodium salts but not into a solution containing calcium salts.

In some embodiments, the compressible foams described herein may be constituted of at least one biocompatible polymeric foaming agent and at least one biocompatible plasticizer.

In some embodiments, the compressible foams described herein may be constituted of at least one biocompatible polymeric foaming agent and at least one biocompatible additive.

The compressible foams described herein may be constituted of at least one biocompatible polymeric foaming agent, at least one biocompatible plasticizer, and at least one biocompatible additive.

A biocompatible polymeric foaming agent is configured to aid in forming a foam from an aqueous solution as provided in more detail hereinbelow. Some suitable non-limiting examples of foaming agents include proteins such as gelatins, collagens, poloxamers, polysorbates, methylcellulose (MC), hydroxypropyl methylcellulose (HPMC), hydroxypropyl cellulose (HPC), hydroxyethyl cellulose (HEC) and mixtures thereof.

In some embodiments, the compressible foams described herein may include a biocompatible polymeric foaming agent such as hydroxypropyl methylcellulose. In some embodiments, the hydroxypropyl methylcellulose may have a methyl substitution degree (DS_{methoxy}) ranging from: 1 to 2.5; or 1.5 to 2. In some embodiments, the hydroxypropyl methylcellulose may have a hydroxypropyl molar substitution (MS_{hydroxypropoxyl}) ranging from: 0.1 to 1; or 0.2 to 0.5.

In some embodiments, the hydroxypropyl methylcellulose may have a DS_{methoxy} ranging from 1 to 2.5 and MS_{hydroxypropoxyl} ranging from 0.1 to 1. In some embodiments, the hydroxypropyl methylcellulose may have a DS_{methoxy} ranging from 1.5 to 2 and MS_{hydroxypropoxyl} ranging from 0.2 to 0.5.

The addition of at least one biocompatible plasticizer provides flexibility and softness to the dried compressible foam such that compressible foam is soft and pliable. Some non-limiting suitable examples of biocompatible plasticizers include polyhydric alcohols such as glycerol, sorbitol, xylitol, mannitol, propylene glycol, polyethylene glycol, and mixtures thereof. In some embodiments, the compressible foam includes a plasticizer such as glycerol, sorbitol, or both.

The addition of at least one biocompatible additive may provide mechanical strength to the dried compressible foam and/or may serves as thickener (increase viscosity of the solution) during production of the compressible foam. In some embodiments, the additive is a non-polymeric additive. In some embodiments, the additive is a polymeric additive. Some non-limiting suitable examples of biocompatible additives include alginates, pectins, carrageenans, cellulose based derivatives such as cellulose ethers such as methylcellulose (MC), hydroxypropyl methylcellulose (HPMC), hydroxypropyl cellulose (HPC), hydroxyethyl cellulose (HEC), polysaccharides gums such as xanthan gum and guar gum, dextrans, hyaluronans, pullulans, maltodextrins, gelatins, collagens, polyvinyl-alcohol and polyethylene-glycol, and mixtures thereof.

In some embodiments, the compressible foam includes an additive such as hydroxypropyl methylcellulose. In some embodiments, the hydroxypropyl methylcellulose may have a methyl substitution degree (DS_{methoxy}) ranging from: 1 to 2.5; or 1.5 to 2. In some embodiments, the hydroxypropyl methylcellulose may have a hydroxypropyl molar substitution (MS_{hydroxypropoxyl}) ranging from: 0.1 to 1; or 0.2 to 0.5.

In some embodiments, the hydroxypropyl methylcellulose may have a DS_{methoxy} ranging from 1 to 2.5 and MS_{hydroxypropoxyl} ranging from 0.1 to 1. In some embodiments, the hydroxypropyl methylcellulose may have a DS_{methoxy} ranging from 1.5 to 2 and MS_{hydroxypropoxyl} ranging from 0.2 to 0.5.

### Composite Implant

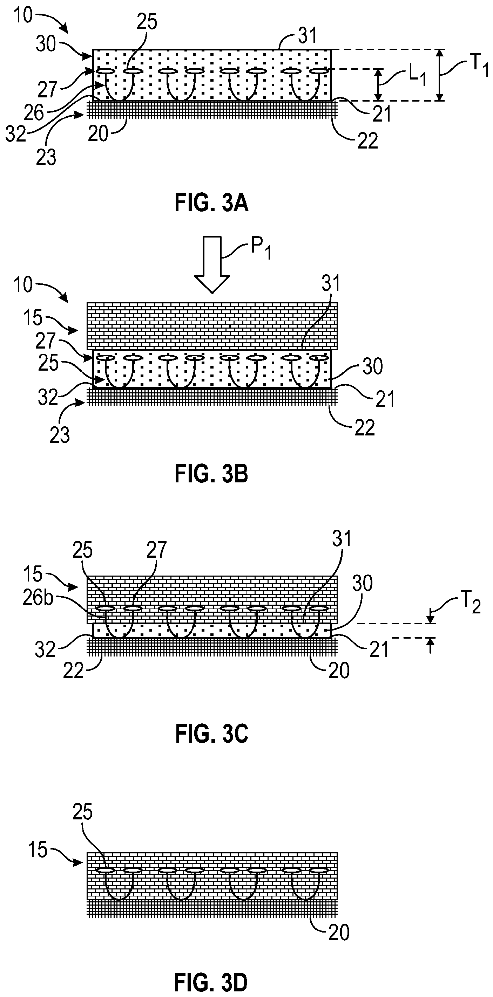

FIG. 3A depicts a schematic cross-sectional view of a composite surgical implant 10 including at least an implantable mesh 20 and a compressible foam 30. The compressible foam 30 covers at least the head portion 27 of one or more, and particularly a plurality of grip members 25. The foam 30 covers the grip member 25, and particularly at least the head portion 27, thereby preventing the grip member 25 from attaching to tissue and/or becoming entangled with other portions of the mesh 20 when overlapped.

In FIG. 3A, the compressible foam 30 is shown in a first initial expanded or non-compressed foam configuration. In the first configuration, the foam 30 may define a generally planar or flat outer top surface 31. In the first configuration, the grip members 25 may be sufficiently buried in the foam 30 such that the grip members 25 are hidden and/or concealed by the foam 30 from at least a top view of the implant 10. In the first configuration, the grip members 25 are in a non-gripping or inactive configuration.

As further shown in FIG. 3A, in the first configuration, the foam 30 defines generally a first thickness T₁ and the grip members 25 define a length L₁. In some embodiments, the foam 30 in the first configuration has a first thickness T₁ greater than or equal to a length L₁ of the grip member 25.

As further depicted in FIG. 3A, in some embodiments, the compressible foam 30 may abut the first face 21 of the mesh body 23 and/or may cover at least an entire length L₁ of the grip body 26 extending or protruding from the first face 21.

FIG. 3B depicts a schematic cross-sectional view of the composite surgical implant 10 of FIG. 3A after a force or pressure P₁, as indicated by the arrow in FIG. 3B, has been applied to the compressible foam 30. As illustrated, the compressible foam 30 is in a second subsequent and/or compressed configuration defining generally a second subsequent thickness T₂ which is less than first thickness T₁. The compressible foam 30 maintains the second configuration even after the force or pressure P₁ is removed (and prior to any dissolution of the foam 30 in vivo).

In some embodiments, as indicated by the arrow in FIG. 3B, the force or pressure P₁ may be applied directly to a first outer surface 31 of the foam 30 in a direction generally towards the mesh body 23 (and/or the first or second mesh face 21, 22). Although the arrow depicts the force or pressure P₁ being applied generally perpendicular to the foam 30 (and/or the first outer surface 31), it is envisioned that in some embodiments the force or pressure P₁ may alternatively be applied at any angle relative to the first outer surface 31 of the foam 30. Some non-limiting examples include the pressure being applied at an angle ranging from 45 to 135 degrees and/or being slid generally parallel to and directly across at least some portion of the first outer surface 31 of the foam 30.

As depicted in FIG. 3B, in the second configuration, the foam 30 may define a generally planar or flat outer top surface 31 and/or the grip members 25 may be sufficiently buried in the foam 30 such that the grip members 25 remain hidden and/or concealed (i.e.,not exposed directly or indirectly) by the compressed foam 30 in the second configuration (from at least a top view of the implant 10). In the second configuration, the grip members 25 may remain in an inactive non-gripping configuration because the compressed foam 30 may still cover at least the head portions 27 of a majority, if not all, of the grip members 25 preventing the head portions 27 from being able to grip to tissue and/or other portions of a mesh. Upon removal of the compressible foam, the grip members 25 transition to an active gripping configuration (FIG. 3C).

In some embodiments, the composite implants 10 described herein may include a compressible foam 30 defining a generally planar or flat outer top surface 31 in both a first expanded foam configuration and second compressed foam configuration (FIGS. 3A-3B).

In some embodiments, the composite implants 10 described herein may include a compressible foam 30 configured to hide or conceal the gripping members 25 within the foam 30 in both a first expanded foam configuration and second compressed foam configuration (FIGS. 3A-3B).

In some embodiments, the composite implants 10 described herein may include a compressible foam 30 configured to render the gripping members 25 in an inactive non-gripping configuration in both the first expanded foam configuration and the second compressed foam configuration (FIGS. 3A-3B). In such embodiments, the gripping members 25 may be transformed into an active gripping configuration after the compressible or compressed foam 30 surrounding at least the head portions 27 of the grip members 25 is removed and/or dissolved away from at least the head portions 27, if not the entire grip member 25. In the active gripping configuration, at least the head portion 27 of the grip member 25 can attach directly to the tissue 15 (and/or other portions of mesh 20) when placed in contact therewith.

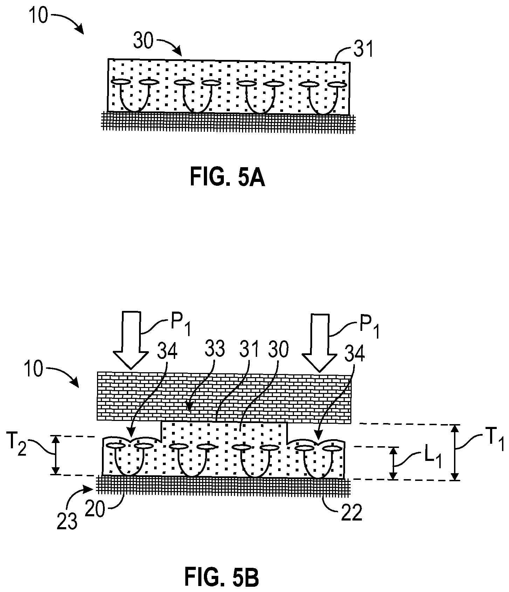

FIG. 3B further depicts that in some embodiments the compressible foam 30 may be compressed evenly across the entire top surface 31, mesh body 23 (and/or the first face 21) and/or the grip members 25 in the second configuration. However, in some embodiments, the foam 30 can be transitioned into the second compressed configuration on only limited portions of the top surface 31, the mesh body 23 (and/or the first or second face 21, 22) and/or only certain grip members 25 (see FIG. 5B).

FIG. 3C depicts a schematic cross-sectional view of the composite surgical implant 10 of FIGS. 3A-3B gripping directly to tissue 15 via the grip members 25, and particularly the second free end portion 26b and/or the head portion 27 of the grip member 25. The compressible foam 30 is also shown positioned between the tissue 15 and the mesh body 23 (and/or first face 21 of the mesh) following dissolution of at least a portion of the foam 30 covering the head portion 27 of the grip members 25. 21

In FIG. 3D, the foam 30 is no longer depicted between the tissue 15 and the mesh body23 (and/or the first face 21) because a majority, if not all, of the foam 30, from FIGS. 3A-3C, dissolved in the physiologic fluids found in the tissue 15. The compressible foam 30 in the second compressed configuration dissolves faster than the compressible foam 30 in the first expanded configuration.

In some embodiments, as depicted in FIG. 3D, the compressible foam 30, in at least the second configuration and optionally the first configuration, may be configured to substantially dissolve, i.e., greater than 90% (if not 100%) of the compressible foam, in physiologic fluids in less than 30 minutes, and in some embodiments less than 15 minutes. In some embodiments, the foam 30 substantially dissolves in physiologic fluids in less than 5 minutes.

In still other embodiments, the compressible foam 30, at least in the second configuration and optionally the first configuration, may substantially dissolve in physiologic fluids from 1 second to 30 minutes. In still other embodiments, the foam 30, at least in the second configuration and optionally the first configuration, may substantially dissolve in physiologic fluids from 5 seconds to 15 minutes. In still other embodiments, the foam 30, at least in the second configuration and optionally the first configuration, substantially dissolves in physiologic fluids from 15 seconds to 10 minutes. In still other embodiments, the foam 30, at least in the second configuration and optionally the first configuration, substantially dissolves in physiologic fluids from 30 seconds to 5 minutes.

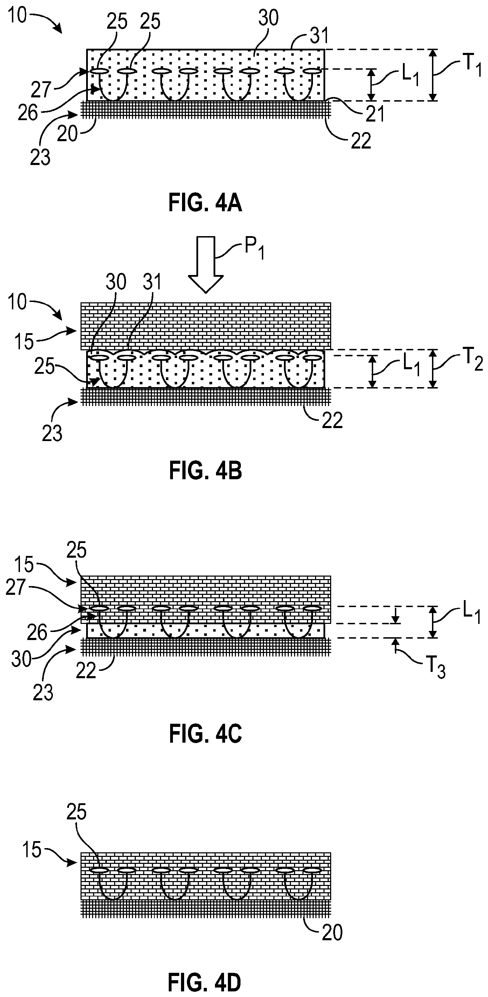

Turning to FIGS. 4A-4D, a composite implant 10 is shown including a mesh 20 and a compressible foam 30 configured to transition from a first configuration (FIG. 4A) to a second configuration (FIG. 4B) before attaching to tissue 15 (FIGS. 4C-4D).

In FIG. 4A, the compressible foam 30 is shown in a first initial expanded or non-compressed configuration. In the first configuration, the foam 30 may define a generally planar or flat outer top surface 31. In the first configuration, the grip members 25 may be sufficiently buried in the foam 30 such that the grip members 25 are hidden and/or concealed by the foam 30 from at least a top view of the implant 10. In the first configuration, the grip members 25 are in an inactive non-gripping configuration.

As further shown in FIG. 4A, in the first configuration, the foam 30 defines generally a first thickness T₁ and the grip members 25 define a length L₁. In some embodiments, the foam 30 in the first configuration has a first thickness T₁ greater than or equal to a length L₁ of the grip member 25.

FIG. 4B depicts a schematic cross-sectional view of the composite surgical implant 10 of FIG. 4A after a force or pressure P₁, as indicated by the arrow in FIG. 4B, has been applied to the compressible foam 30. As shown in FIG. 4B, in some embodiments, the foam 30 in the second compressed configuration follows the outer contour of the head portions 27 of the grip members 25. By following the outer contour of the head portions 27, the foam 30 in the second compressed configuration may display an uneven texture indicative of the general location of grip members 25 beneath the compressed foam 30. In the second configuration, the grip members 25 may remain in a non-gripping or inactive configuration because the compressed foam 30 may still cover at least the head portions 27 of a majority, if not all, of the grip members 25 preventing the head portions 27 from being able to grip to tissue and/or other portions of a mesh.

In some embodiments, the composite implants 10 described herein may include a compressible foam 30 defining a generally planar or flat outer top surface 31 in a first expanded foam configuration and an uneven texture in a second compressed foam configuration (FIGS. 4A-4B).

In some embodiments, the composite implants 10 described herein may include a compressible foam 30 configured to hide or conceal the gripping members 25 within the foam 30 in a first expanded foam configuration and indirectly expose the gripping members 25 in the second compressed foam configuration (FIGS. 4A-4B).

In some embodiments, the composite implants 10 described herein may include a compressible foam 30 configured to render the gripping members 25 non-gripping or inactive in both the first expanded foam configuration and the second compressed foam configuration (FIGS. 4A-4B). In such embodiments, the gripping members 25 may be transformed into a gripping or active configuration after the compressible or compressed foam 30 surrounding at least the head portions 27 of the grip members 25 is removed and/or dissolved away from at least the head portions 27, if not the entire grip member 25. In the gripping or active configuration, at least the head portion 27 of the grip member 25 can attach directly to the tissue 15 (and/or other portions of mesh 20) when placed in contact therewith.

FIG. 4C depicts a schematic cross-sectional view of the composite surgical implant 10 of FIGS. 4A-4B gripping directly to tissue 15 via the grip members 25, and particularly the second free end portion 26b and/or the head portion 27 of the grip member 25. The compressible foam 30 is also shown positioned between the tissue 15 and the mesh body 23 (and/or first face 21 of the mesh) following dissolution of at least the portion of the compressed foam 30 covering the head portion 27 of the grip members 25.

In FIG. 4D, the foam 30 is no longer depicted between the tissue 15 and the mesh body23 (and/or the first face 21) because a majority, if not all, of the foam 30, from FIGS. 4A-4C, dissolved in the physiologic fluids found in the tissue 15. The compressible foam 30 in the second compressed configuration dissolves faster than the compressible foam 30 in the first expanded configuration.

Turning to FIGS. 5A-5B, in some embodiments, a composite implant 10 is shown including a mesh 20 and a compressible foam 30 configured to transition from a first expanded configuration (FIG. 5A) to a second compressed configuration (FIG. 5B). In FIG. 5A, the composite implant 10 is similar to the composite implants described herein, and particularly described in FIGS. 3A and 4A. In FIG. 5B, the foam 30 is shown in a second compressed configuration.

FIG. 5B depicts a schematic cross-sectional view of the composite surgical implant 10 of FIG. 5A after a force or pressure P₁, as indicated by the arrows in FIG. 5B, has been applied to the compressible foam 30 on opposite ends of the foam 30 and/or mesh 20. As depicted in FIG. 5B, in some embodiments, the foam 30 may simultaneously include one or more first zones 33 in a first expanded configuration and one or more second zones 34 in a second compressed configuration. In some embodiments, a central first zone 33 may be positioned between a pair of outer second zones 34. Any combination of first and second zones 33, 34 may be combined across a portion, if not an the entire, foam and/or mesh. The foam can be removed or dissolved as described herein to activate the grip members to grip tissue and/or other portions of a mesh body.

Although the second zones 34 are shown in FIG. 5B to follow the contour of the head portions 27 of the grip members 25 to produce an uneven textured outer foam surface along the outer surface 31 of the second zones 34, in some embodiments, at least one of the two second zones of FIG. 5B may include a generally planar or flat outer top surface, like the first zone (similar to as shown in FIG. 3B).

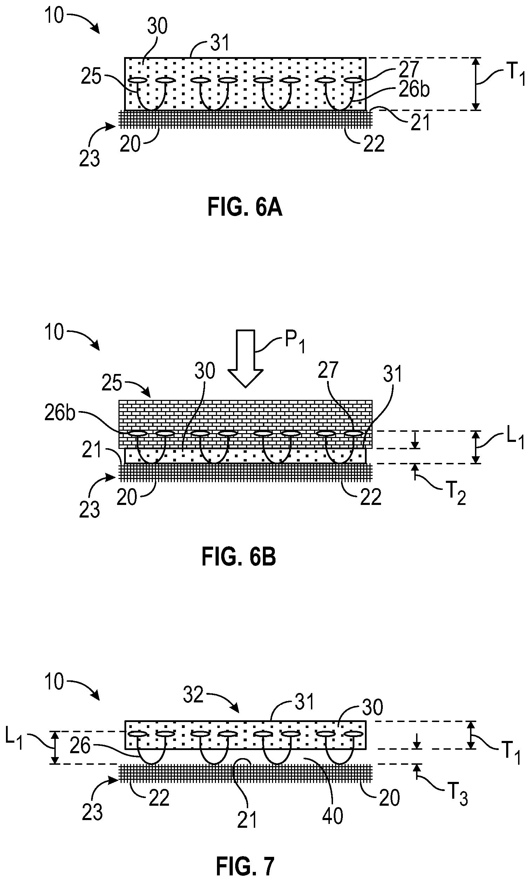

Turning to FIGS. 6A-6B, in some embodiments, a composite implant 10 is shown including a mesh 20 and a compressible foam 30 configured to transition from a first expanded configuration (FIG. 6A) to a second compressed configuration (FIG. 6B). In FIG. 6A, the composite implant 10 is similar to the composite implants described herein, and particularly described in FIGS. 3A and 4A. In FIG. 6B, the foam 30 is shown in a second compressed configuration.

FIG. 6B depicts a schematic cross-sectional view of the composite surgical implant 10 of FIG. 6A after a force or pressure P₁, as indicated by the arrows in FIG. 6B, has been applied to the compressible foam 30. As illustrated, the compressible foam 30 is in a second subsequent and/or compressed configuration defining generally a second subsequent thickness T₂ which is less than first thickness T₁ and/or less than the length L₁ of the grip member 25. In some embodiments, the foam 30 in the second configuration exposes at least the head portion 27, and possibly the distal end portion 26b, of the grip member 25 thereby allowing the grip member 25 to attach directly to the tissue 15 (and/or other portions of mesh 20) when placed in contact therewith. In such embodiments as shown in FIGS. 6A-6B, the compressible and/or compressed foam 30 does not need to be dissolved for the grip members 25 to be active because the grip members 25 are directly exposed and extend beyond the outer surface 31 of the foam 30 in the compressed second configuration.

Turning to FIG. 7, in some embodiments, the compressible foam 30, in the first initial or non-compressed configuration, may have a first thickness T₁ lesser than or equal to a length l₁ of the gripping element 25. In such embodiments, a gap 40 may exist between the compressible foam 30, and particularly the second inner surface 32 of the compressible foam 30 and the mesh body 23 (and/or the first face of the mesh 21). The gap 40 defines a gap thickness T₃ generally along the portion of the grip body 26 which is not covered by the foam 30. In some embodiments, the gap thickness T₃ and the first thickness T₁ of the compressible foam in the first configuration are equal. In some embodiments, the gap thickness T₃ is less than the first thickness T₁ of the compressible foam in the first configuration.

It is envisioned that the gap 40 may enhance the ability of the implant 10 or mesh 20 to incorporate or promote tissue growth therein. In addition, the gap 40 reduces the amount of compressible foam implanted with the composite implant thereby reducing the amount of foreign material incorporated into the overall composite implant. It is further envisioned that the gap 40 reduces the resorption time of the compressible foam in vivo.

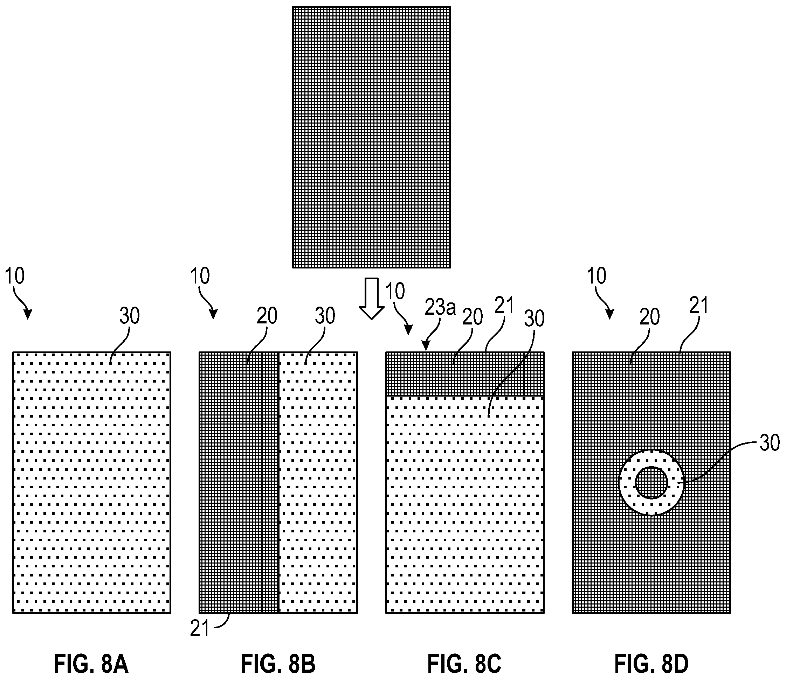

FIGS. 8A-8D provide some non-limiting examples of a generally rectangular composite implant 10 including an implantable mesh 20 and a compressible foam 30 in various designs. For example, FIG. 8A depicts embodiments wherein a compressible foam 30 is positioned on the grip members across an entirety of a first face 21 of a mesh 20. In another example, FIG. 8B depicts embodiments wherein a compressible foam 30 is positioned on the grip members across only a first half of a first face 21 of a mesh 20 thereby leaving a second half of the first face 21 of the mesh 20 uncovered by the foam 30. In still another example, FIG. 8C depicts embodiments wherein a compressible foam 30 is positioned on the grip members across more than a majority of a first face 21 of a mesh 20 and leaving a band along an outer edge 23a of the mesh 20 uncovered by the foam 30. In yet another example, FIG. 8D depicts embodiments wherein a compressible foam 30 positioned on the grip members may form a particular given shape, such as a ring (or a letter, a number, a polygon, etc.) on a portion of the first face 21 of a mesh 20, and particularly a central portion of the first face 21. It is envisioned that combinations and/or variations of the implants of FIGS. 8A-8D are also possible.

In each of FIGS. 8A-8D, the grip members are positioned on at least the first portions of the first face 21 of the mesh 20 that the foam is located, i.e., beneath the foam 30. In addition, the grip members 25 may or may not also be positioned on the second uncovered portions of the first face 21 of the mesh 20 in FIGS. 8A-8D.

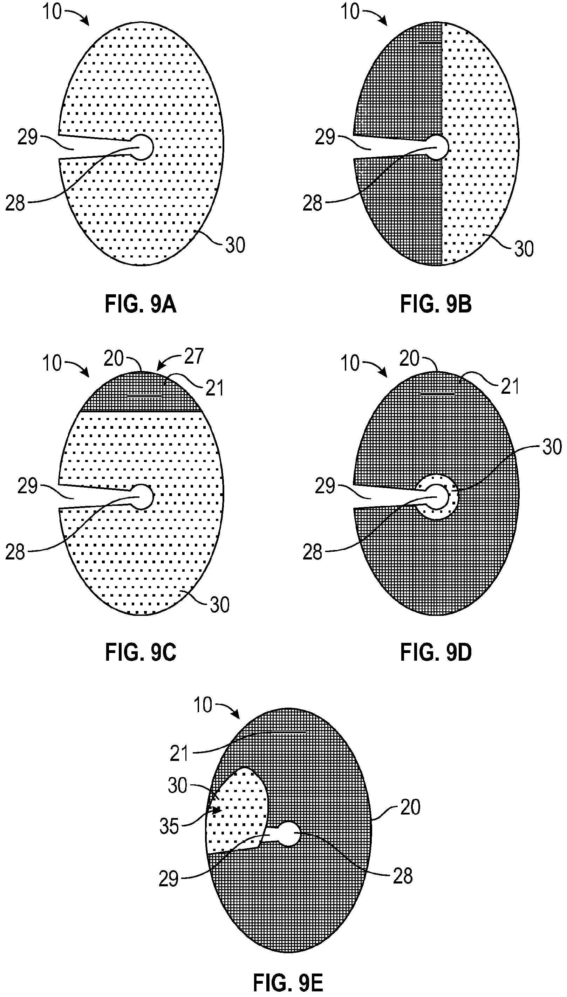

FIGS. 9A-9E provide some non-limiting examples of a generally elliptical composite implant 10 including an implantable mesh 20 with a central opening 28 therethrough and a slit 29, and a compressible foam 30 in various designs. An overlapping flap mesh is not shown in FIGS. 9A-9D for clarity purposes but may be positioned on a face of the mesh 20 and extending across a portion of the slit 29, as shown in FIG. 9E.

FIG. 9A depicts embodiments wherein a compressible foam 30 is positioned on the grip members across an entirety of a first face 21 of a mesh 20. FIG. 9B depicts embodiments wherein a compressible foam 30 is positioned on the grip members across generally a first half of a first face 21 of a mesh 20 thereby leaving generally a second half of the first face 21 of the mesh 20, including the central opening 28 and the slit 29, uncovered by the foam 30. FIG. 9C depicts embodiments wherein a compressible foam 30 is positioned on the grip members across more than a majority of a first face 21 of a mesh 20 (including along an edge of the central opening 28 and the slit 29) and leaving a band along an outer edge 37 of the mesh 20, uncovered by the foam 30. FIG. 9D depicts embodiments wherein a compressible foam 30 positioned on the grip members may be positioned along an edge of the central opening 28 and not along an edge of the slit 29.

FIG. 9E depicts embodiments wherein the composite implant 10 includes an implantable mesh 20 with a central opening 28 therethrough and a slit 29, an overlapping flap mesh 35 configured to extend across at least the slit 29, and a compressible foam 30. The compressible foam 30 is positioned between the first face 21 of the mesh 20 and an inner surface of the flap mesh 35. In some embodiments, the grip members and the compressible foam 30 may be positioned on the first face of the mesh 21 while the inner surface of the flap is free of the grip members and/or the foam. In other embodiments, the grip members and the compressible foam 30 may be positioned on the inner face of the flap mesh 35 while the first face 21 of the mesh 20 is free of the grip members and/or the foam.

It is envisioned that combinations and/or variations of the implants of FIGS. 9A-9E are also possible. In each of FIGS. 9A-9E, the grip members 25 are positioned on at least the first portions of the first face 21 of the mesh 20 that the foam 30 is located, i.e., beneath the foam. In addition, the grip members 25 may or may not also be positioned on the second uncovered portions of the first face 21 of the mesh 20 in FIGS. 9A-9E.

Only partially covering the face 21 of the mesh 20 with the compressible foam 30 provides several advantages, including improvement of the handling of the composite implant without having to change any surgical practices by the medical staff or surgeon. For example, in order to prevent sliding of an implantable mesh during its deployment on tissues, the medical staff or surgeon may start the surgical procedure by adding a stich to the mesh to anchor one portion of the mesh to the tissue while trying to properly orient the rest of the mesh. By leaving a portion of the face of the mesh uncovered with the compressible foam 30 (FIGS. 8B-8D and 9B-9E), the medical staff or surgeon maintains the ability to add a stitch directly to the uncovered portion of the face 21 of the mesh 20 without damaging or piercing the compressible foam 30, and with a much better visibility, thus limiting the risk of trapping some critical anatomical structures like nerves.

Moreover, not covering a specific portion provides the possibility to add a separate orientation marker or marking means on the uncovered portion of the mesh. For example, as shown in FIGS. 8C and 9C, an uncovered or non-foamed band of mesh along an outer edge of the mesh may be configured to be positioned on the pubis. So, leaving the band of the mesh uncovered by the foam can provide an orientation marking to the medical staff or surgeon to help facilitate the proper insertion and deployment of the mesh.

In another example, the medical staff or surgeons may be concerned with trying to avoid or prevent damage to the spermatic cord by the grip members and/or the lateral edges of the mesh during orientation of the mesh, because such damage can lead to severe post-operative pains. By selectively incorporating the compressible foam onto various combinations of grip members, it is possible to selectively cover the grip members located on the mesh which are designed to come into contact with the spermatic cord, such as around a central opening and/or along a slit of the mesh (FIGS. 9A, 9C, 9D) or to reinforce the foam coverage of this part to prevent potential damages and pains.

### Methods of Manufacture

The composite implants described herein include at least an implantable mesh and a compressible foam, each of which may be manufactured using a variety of methods.

As noted hereinabove, the implantable mesh (and/or a flap mesh) may be formed using any suitable method known to those of ordinary skill including, but not limited to, weaving, knitting, braiding, crocheting, embroidering, and the like. In some embodiments, the implantable mesh (and/or flap mesh) may be formed by knitting to form a knit mesh. In some embodiments, the implantable mesh (and/or flap mesh) may be made as provided in any of the U.S. Patent Nos. 6,596,002; 7,331,199; 9,750,595; 9,186,235; 9,510,927.

As further noted hereinabove, the compressible foam as described herein is in a dry or dried format. The compressible foam is derived from a wet non-flow foam material prior to drying. By "non-flow," the wet foam material has a viscosity high enough to prevent the wet foam from flowing or running when applied to the implantable mesh, prior to drying. The wet non-flow foam material may be formed from a solution as described herein. The solution may have a dynamic viscosity (at zero shear) ranging from 10mPa.s⁻¹ to 1000Pa.s⁻¹; 100mPa.s⁻¹ to 100Pa.s⁻¹; or 1Pa.s⁻¹ to 50Pa.s⁻¹s.

Methods of forming a compressible foam include first forming a wet non-flow foam material followed by drying the wet non-flow foam material to form the compressible foam (in dry form).

To form or produce the wet non-flow foam material (and/or the dried compressible foam) of the present disclosure, at least one biocompatible polymeric foaming agent, which is soluble within physiological conditions, is combined with a pharmaceutical carrier to form a solution. The solution may optionally include one or more biocompatible plasticizers and/or one or more biocompatible additives, each of which are also soluble within physiological conditions or similar fluids such as saline. In addition, the solution may further include one or more bioactive agents.

The one or more biocompatible polymeric foaming agents may represent from: 0.01 to 25 weight percent; 0.1 to 20 weight percent; 0.5 to 15 weight percent; or 1 to 10 weight percent, of the solution.

Some non-limiting examples of suitable solvents include water (e.g., sterile water, non-sterile water, distilled water, spring water, etc.), saline, dextrose solution, and the like. In some embodiments, the solvent is a water and the solution is an aqueous solution.

Some suitable non-limiting examples of the one or more biocompatible polymeric foaming agents includes proteins such as gelatin, poloxamers, polysorbates, methylcellulose (MC), hydroxypropyl methylcellulose (HPMC), hydroxypropyl cellulose (HPC), hydroxyethyl cellulose (HEC) and mixtures thereof. In some embodiments, the biocompatible polymeric foaming agent is hydroxypropyl methylcellulose. The hydroxypropyl methylcellulose may have a methyl substitution degree (DS_{methoxy}) ranging from 1 to 2.5 or 1.5 to 2. The hydroxypropyl methylcellulose may have a hydroxypropyl molar substitution (MS_{hydroxypropoxyl}) ranging from 0.1 to 1 or 0.2 to 0.5. In some embodiments, the hydroxypropyl methylcellulose may be combined with a water to form an aqueous solution. The dynamic viscosity of the hydroxypropyl methylcellulose may represent from 1 to 100mPa.s⁻¹; or 3 to 50mPa.s⁻¹, measured as a 2 weight% aqueous solution at 20°C using an Ubbelohde-type viscometer.

Some suitable non-limiting examples of the one or more plasticizers include polyhydric alcohols such as glycerol, sorbitol, xylitol, mannitol, propylene glycol, polyethylene glycol, and mixtures thereof. In some embodiments, the solution may include a plasticizer such as glycerol, sorbitol, or both. When incorporated into the solution, the plasticizer may represent from: 0.5 to 40 weight percent; 1 to 20 weight percent; or 2 to 10 weight percent, of the solution.

Some suitable non-limiting examples of the one or more biocompatible additives include alginates, pectins, carrageenans, cellulose based derivatives such as cellulose ethers such as methylcellulose (MC), hydroxypropyl methylcellulose (HPMC), hydroxypropyl cellulose (HPC), hydroxyethyl cellulose (HEC), polysaccharides gums such as xanthan gum and guar gum, dextrans, hyaluronans, pullulans, maltodextrins, gelatin, polyvinyl-alcohol and polyethylene-glycol, and mixtures thereof. In some embodiments, the solution may include a biocompatible additive such as hydroxypropyl methylcellulose. The hydroxypropyl methylcellulose may have a methyl substitution degree (DS_{methoxy}) ranging from 1 to 2.5 or 1.5 to 2. The hydroxypropyl methylcellulose may have a hydroxypropyl molar substitution (MS_{hydroxypropoxyl}) ranging from 0.1 to 1 or 0.2 to 0.5. The viscosity of the hydroxypropyl methylcellulose may represent from 1 to 100mPa.s⁻¹; or 3 to 50mPa.s⁻¹, measured as a 2 weight % aqueous solution at 20°C using an Ubbelohde-type viscometer. When incorporated into the solution, the additive may represent from: 0.1 to 10 weight percent; 0.5 to 5 weight percent; or 1 to 3 weight percent, of the solution.

Bioactive agents include substances which are beneficial and tend to promote the healing process. Suitable examples of bioactive agents include, for example, biocidal agents, antimicrobial agents, antibiotics, anti-proliferatives, medicants, growth factors, anti-clotting agents, clotting agents, analgesics, anesthetics, anti-inflammatory agents, wound repair agents and the like, chemotherapeutics, biologics, protein therapeutics, monoclonal or polyclonal antibodies, DNA, RNA, peptides, polysaccharides, lectins, lipids, probiotics, diagnostic agents, angiogenics, anti-angiogenic drugs, polymeric drugs, and combinations thereof. In some embodiments, the bioactive agent may represent less than 10% and particularly less than 5% of the solution.

After combining the polymeric foaming agent, and optionally at least one of the plasticizer or additive, with the carrier to form the solution, air may be mechanically or physically entrained or dispersed into the solution to form a wet foam material including a first discontinuous air phase, and a second continuous aqueous phase. The lamella or fluid film of the air bubbles entrained or dispersed within the wet foam material is viscous due to the presence of the polymeric foaming agent. The flow of any liquid retained in the lamella of the air bubbles is minimized, reduced or prevented in the obtained wet foam material and as such, the wet foam material can be described as "non-flow". The wet non-flow foam material have a density ranging from about 0.05 g.cm⁻³ to 0.50 g.cm⁻³; 0.10 g.cm⁻³ to 0.40 g.cm⁻³; or 0.20 g.cm⁻³ to 0.30 g.cm⁻³.

Advantageously, this "non-flow" property of the wet foam material offers the possibility to cover the grip members, in various shapes, designs, or thicknesses, using a variety of different manners including but not limited to at least one of casting, molding, rolling, screening, dropping with a utensil such as a syringe, spoon, spatula, brush, sponge, roller, and the like. For example, it is possible to cover (or not cover) all or only selected portions of a face of the implantable self-fixating mesh with the wet non-flow foam material and/or dry compressible foam.

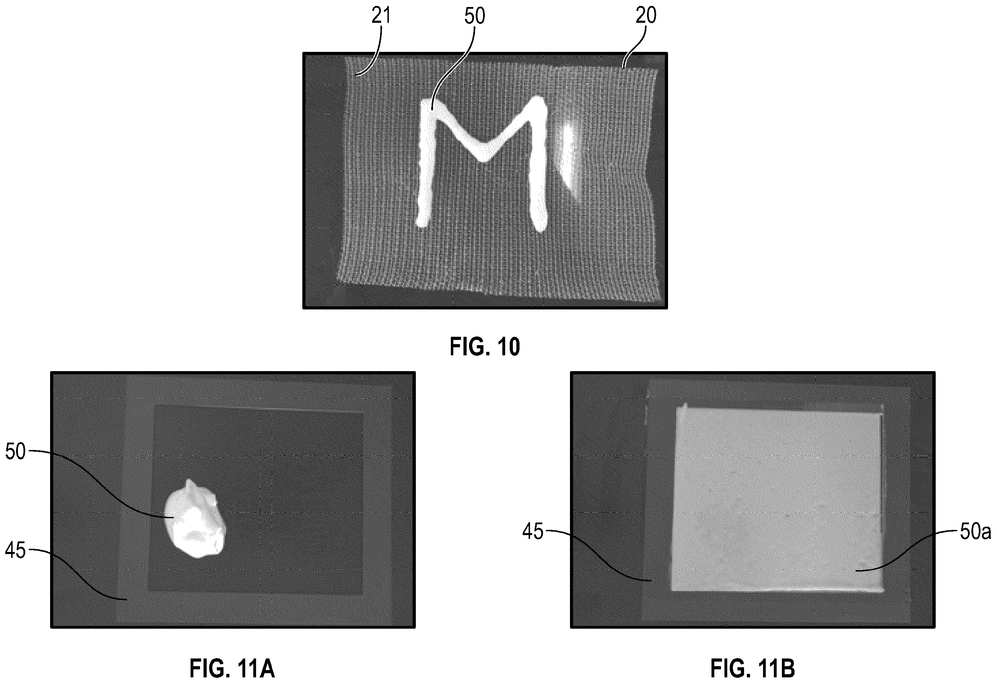

The wet non-flow foam material is configured to be applied to the implantable mesh either directly or indirectly. As illustrated in FIG. 10, in some embodiments, the wet non-flow foam material 50 can be applied directly to the grip members of the implantable mesh 20. For example, in FIG. 10, the wet non-flow foam material 50 is depicted on the grip members of the mesh 20 in the pattern, such as the shape of a letter "M". Due to the viscosity of the non-flow wet foam material, the wet foam can be applied directly to the mesh in any variety of patterns including but not limited to, stripes, polygons, circles, ovals, rings, dots, dashes, arrows, numbers, letters, tear drops, etc. The wet non-flow foam material can be directly applied to grip members of the mesh using any suitable utensil, including but not limited to a syringe, spoon, fork, ladle, scoop, straw, brush, roller, and/or piping bag. Once applied, the pattern is maintained through the drying process to produce a dry compressible foam of a same or similar pattern.

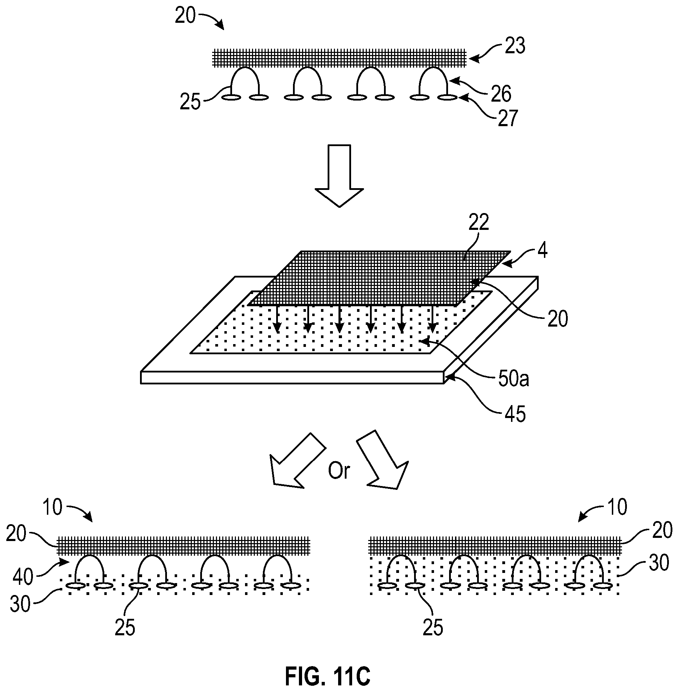

FIGS. 11A-11C depict methods of manufacturing a composite implant wherein the wet non-flow foam material is applied to the mesh indirectly. As illustrated, the methods include: applying the wet non-flow foam material 50 to a frame or mold 45 (FIG. 11A); casting and/or leveling of the wet non-flow foam material 50 directly across a top 46 of the mold or frame 45 to define a wet non-flow foam layer 50a of a consistent thickness equal to the thickness of the mold or frame 45 (FIG. 11B); and placing the grip members 25 of the implantable mesh 20 into the leveled wet non-flow foam layer 50a; and drying the wet foam layer 50a to form a dry compressible foam 30 on the grip members 25 of the mesh 20 to form a composite implant 10 (FIG. 11C).

FIG. 11A depicts an initial step of applying the wet foam to the frame or mold. For example, one or more dollops 51 of the wet non-flow foam material 50 can be placed into the mold or frame 45. The volume of wet non-flow foam may be larger than the volume of the frame. The frame or mold may be positioned on a level surface.

FIG. 11B depicts the wet non-flow foam layer 50a cast and leveled directly across the top 46 of the mold or frame 45 to define a generally planar wet non-flow foam layer 50a of a consistent thickness equal to the thickness of an inert and/or non-stick mold or frame 45. Any suitable method of casting and/or leveling of the wet foam layer may be used including but not limited to stamping, molding, spreading, pressing, and the like.

FIG. 11C depicts specifically that the mesh 20 may be lowered onto the top layer of the wet non-flow foam layer 50a with the grip members 25 facing downward and leading the way towards the layer 50a of the wet foam with the mesh body 23 trailing behind the grip members 25. The mesh body may or may not enter the wet non-flow layer.

Because the wet non-flow foam material is leveled to the top of the frame or mold, the thickness of the frame or mold can be used to define the thickness of the wet non-flow foam layer and as result, the dry compressible foam. For example, as shown in FIG. 11C, when the frame 45 thickness is less than the length of the body 26 of the grip members 25, the wet foam layer 50a can be cast and leveled to fill the entire frame 45 so that the grip members 25 are only superficially covered thereby creating a gap 40 between the mesh body 23 and the wet non-flow foam layer 50a (and/or the compressible foam 30 when dried). In another example, when the frame 45 thickness is greater than the length of the body 26 of the grip members 25, the wet foam layer 50a can be cast and leveled to fill the entire frame 45 so that the grip members 25 are typically fully covered and the mesh body 23 and the wet non-flow foam layer 50a (and/or the compressible foam 30 when dried) may abut up against each other.

In some embodiments, following the casting and leveling of the wet foam material to form a wet foam layer within the frame, the framed wet foam layer may be warmed to partially harden or dry prior to being combined with the grip members of the implantable mesh. For example, in some embodiments, the framed wet non-flow foam layer may be warmed using an oven, such as a forced air oven. In order to prevent the framed wet foam layer from transitioning completely to the dry compressible foam prior to the addition of the mesh, the framed wet non-flow material can be heated to 50°C for up to 5 minutes prior to the application of the mesh.

After the framed wet non-flow foam layer and the implantable mesh including grip members are combined, the wet non-flow foam layer may be completely dried into the compressible foam by heating the combined wet foam layer and mesh in an oven, such as a forced air oven. The combined wet foam layer and mesh can be heated to 50°C for 2 hours to form a dry compressible foam on the mesh forming a composite implant.

The compressible foam surface density on the composite implant may range from about: 0.1mg/cm² to 10mg/cm²; 0.5mg/cm² to 5mg/cm²; or 1mg/cm² to 3mg/cm².

The compressible foam may represent from about 5% to 50%; 10% to 40%; or 15% to 30% of the implant mass.

Once formed, the composite implants described herein may be sterilized and packaged using an suitable manner known to those skilled in the art.

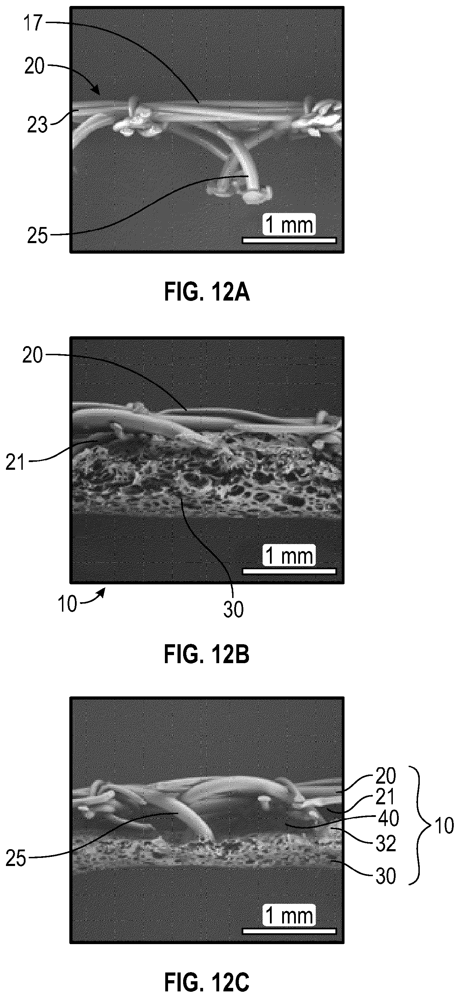

FIG. 12A is an image from a scanned electron microscope depicting one example of a suitable self-fixating implantable mesh 20 prior to being combined with the compressible foam, the implantable mesh 20 including one or more filaments 17 intertwined to form a mesh body 23 and a plurality of grip members 25, such as spiked naps, extending therefrom.

FIG. 12B is an image from a scanned electron microscope depicting one example of a composite implant 10 including an implantable mesh 20 including grip members completely or fully covered with the compressible foam 30, the compressible foam 30 depicted in the first uncompressed configuration having a first initial thickness, prior to compression and abutting up against the face 21 of the mesh 20.

FIG. 12C is an image from a scanned electron microscope depicting one example of a composite implant 10 including an implantable mesh 20 including grip members 25 only partially covered with the compressible foam 30, the compressible foam 30 depicted in the first uncompressed configuration having a first initial thickness, prior to compression. A gap 40 is positioned between the inner second surface of the foam 32 and the face 21 of the mesh 20.

After drying, the composite implants described herein include a mesh having at least some, if not all, grip members covered with a compressible foam in the first initial configuration having a first thickness, the compressible foam being configured to transition to a second configuration having a second thickness smaller than the first thickness.

The compressible foam in the first configuration prevents the covered grip members from attaching to tissue and/or becoming entangled with other portions of the mesh. In some embodiments, the compressible foam in the second compressed configuration may prevent the covered grip members from attaching to tissue and/or becoming entangled with other portions of the mesh. In such embodiments, the composite implant in either of the first or second configurations may be rolled or folded without fear of the grip members attaching to overlapping portions of the rolled mesh and thus remaining configured to unroll without attachment. Also, in such embodiments, activation of the grip members requires the presence of physiologic fluids or saline, water, or the like.

Upon implantation, and exposure to physiologic fluids, the compressible foam in either configuration will begin to hydrate. Following resorption or dissolution of the compressible foam, from either configuration, the gripping performance of the composite implant is generally equal to the gripping performance of the self-fixating mesh prior to being combined with the compressible foam.

In some embodiments, the compressible foam in the second compressed configuration directly exposes at least the head portions of the grip members thereby allowing the grip members to attach directly to tissue and/or become entangled with other portions of the mesh.

The compressible foam also adds rigidity to the mesh which renders the composite implant naturally self-deploying, i.e., unrolling or unfolding. The added rigidity also renders the composite implant generally crinkle-free, i.e., greater than 90%, if not 95% or 100%, of the surface area of the composite implant remains free of entanglements or crinkles when unfolded or unrolled after insertion directly through an incision or through a trocart.

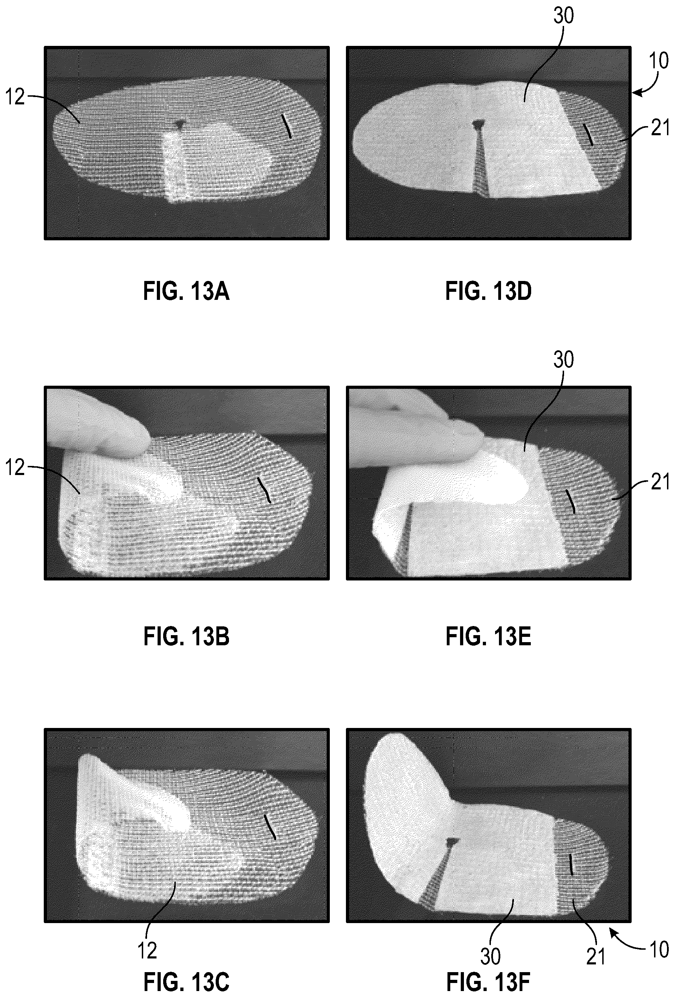

FIGS. 13A-13F illustrate the difficulty of folding and unfolding of a typical self-fixating mesh 12 free of a compressible foam as compared to the ease of folding and unfolding of the composite implants 10 described herein. Specifically, FIGS. 13A-13C illustrate that when the self-fixating mesh 12 free of a compressible foam is forced (e.g., by hand) to fold or roll over itself, the uncovered grip members become entangled with the overlapping portion of the mesh preventing the natural unfolding or unrolling of the mesh upon removal of the force. However, as specifically illustrated in FIGS. 13D-13F, when a composite implant 10 described herein is forced to fold or roll over itself, the foam 30 prevents the grip members from becoming entangled with the overlapping portion of the mesh 20 thereby allowing for a natural self-deploying, i.e., self-unfolding or self-unrolling, of the composite implant 10 when the force is removed.

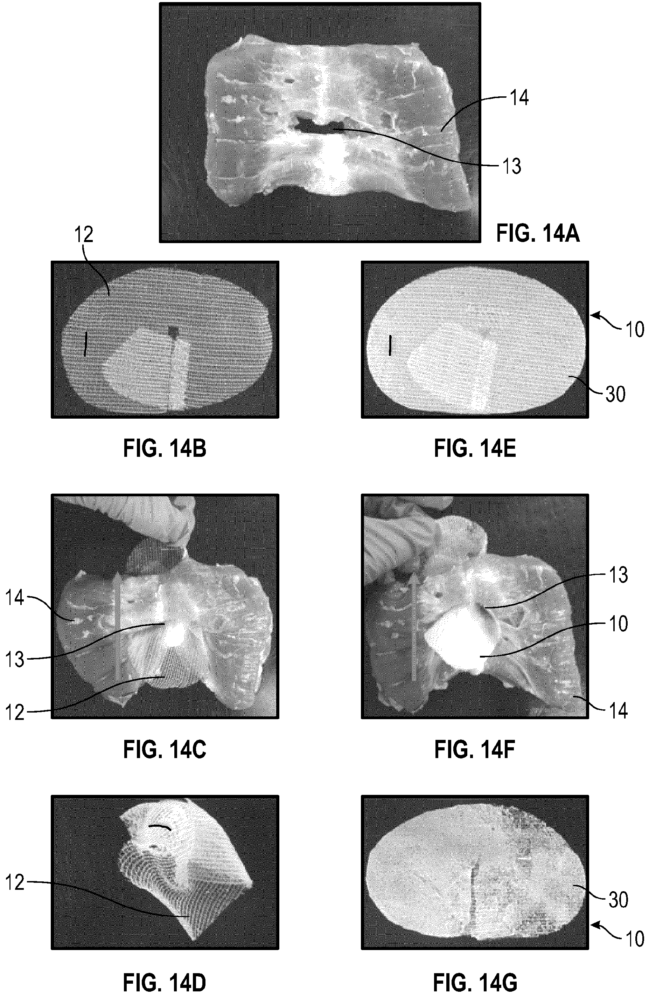

FIGS. 14A-14G illustrate that inactivation of the coated grips, as well as the added rigidity of the composite implants described herein, as compared to a self-fixating mesh free of a compressible foam, renders the composite implant generally entanglement-free, i.e., greater than 90%, if not 95% or 100%, of the surface area of the composite implant remains free of entanglements or crinkles when unfolded or unrolled after insertion through an incision. FIG. 14A displays an incision 13 in animal tissue 14. FIGS. 14B-14D illustrate that following insertion of a folded or rolled self-fixating mesh 12 free of a compressible foam, the self-fixating mesh 12 (free of the compressible foam) remains entangled after insertion through the incision. As shown in FIG. 14D, only about 40-60% of the surface area of the mesh is unfolded or unrolled due to the entanglements of grip members with overlapping portions of the mesh 12. However, as illustrated in FIGS. 14E-14G, following insertion of a folded or rolled composite implant 10 as described herein, the inserted composite implant 10 remains entanglement-free or greater than 90% or 95% of the surface area of the implant 10 is free of entanglements because the compressible foam 30 prevents the grip members from becoming entangled during rolling and unrolling (or folding and unfolding).

In some embodiments, a method of manufacturing a composite implant includes: providing an implantable mesh having one or more grip members extending or protruding therefrom, each grip member configured to attach directly to tissue of a patient and/or configured to become secured or entangled to other portions of the implantable mesh when overlapped; applying a wet non-flow foam material to at least a second free end of each of the one or more grip members, and drying the wet non-flow foam to form a compressible foam in a first configuration having a first thickness, the compressible foam configured to transition from the first configuration having the first thickness to a second configuration having a second thickness smaller than the first thickness. In humid conditions, the second free ends of the plurality of grip members being exposed to attach to tissue when the foam is in the second configuration.

### Methods of Use

The composite implants described herein are useful for the repair or treatment of soft tissue openings or wounds. Some non-limiting examples include hernia repair, prolapse repair, dural repair, ostomy repair, and the like. In some embodiments, the composite surgical implants described herein are useful for hernia repairs selected from ventral hernias, inguinal hernias, umbilical hernias, etc.

The soft tissue repair or treatment may be performed via open or laparoscopic procedure. In some embodiments, the soft tissue repair or treatment may be for a hernia repair via laparoscopic procedure, such as transabdominal preperitoneal (TAPP) repair or totally extraperitoneal (TEP) repair.

In some embodiments, the composite implants may be used in a method of soft tissue repair. The method may include inserting a composite implant into a site of implantation of a patient near a wound in the soft tissue in need of repair, the composite implant including a mesh and compressible foam as described herein having a first face and a second face opposite the first face, a plurality of grip members extending from the first face of the mesh, each of the plurality of grip members including a body extending between a first end attached to the mesh and a second end free of the mesh, and a compressible foam covering at least the second ends of the plurality of grip members, the compressible foam configured to transition from a first configuration having a first thickness to a second configuration having a second thickness smaller than the first thickness, and exposing the second ends of the plurality of grip members. Following insertion, the method further includes: orienting the composite implant across the wound in the soft tissue; applying pressure to a portion of the compressible foam to transition from the first configuration to the second configuration having a second thickness less than the first thickness thereby exposing the second ends of at least some portion of the plurality grip members; and securing the grip members including the compressible foam in the second configuration to the tissue taking benefit of the presence of body fluids.

Prior to inserting the composite implant, the methods of soft tissue repair may further include forming an incision on the patient prior to inserting the composite implant and optionally positioning a trocar in the incision for inserting the composite implant therethrough. In addition, the method may further include rolling or folding the composite implant prior to inserting into the site of implantation and unrolling or unfolding the composite implant after being inserted into the site of implantation.

In some embodiments, the step of applying pressure to a portion of the compressible foam may include applying the pressure: directly to the first outer surface of the compressible foam; indirectly to the second face of the mesh opposite the first face of the mesh including the compressible foam; or both, in contact with biological tissues.

In some embodiments, the step of applying pressure to a portion of the compressible foam may include applying the pressure directly to outer surface of the compressible foam in contact with biological tissues. The pressure may be applied directly to the foam: at an angle generally perpendicular position relative to a longitudinal axis of the compressible foam; at an angle ranging from about 15° to about 75° relative to a longitudinal axis of the compressible foam; or by sliding the pressure across the first outer surface of the compressible foam.

In some embodiments, the step of applying pressure to a portion of the compressible foam in contact with biological tissues may include applying the pressure indirectly to the second face of the mesh opposite the first face of the mesh including the compressible foam: at an angle generally perpendicular position relative to a longitudinal axis of the mesh (or the compressible foam); at an angle ranging from about 15° to about 75° relative to a longitudinal axis of the mesh (or the compressible foam); or by sliding the pressure across the second face of the mesh.

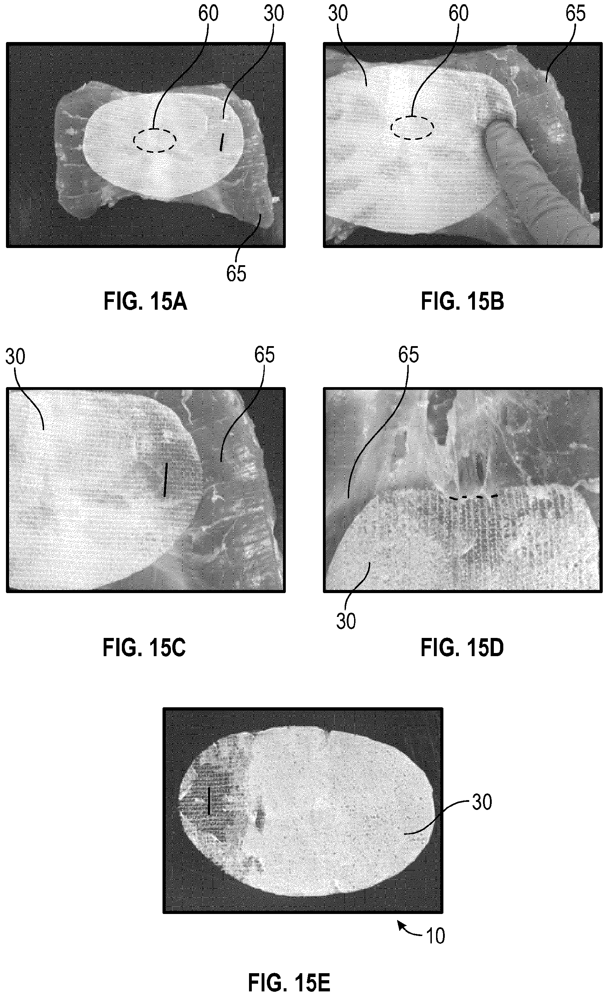

As shown in FIG. 15A, in some embodiments, following insertion, the composite implant 10 can be oriented across a wound or opening 60 in the soft tissue 65 with the compressible foam, in the first configuration, facing the soft tissue 65 and the second opposite face of the mesh 22 facing away from the wound 60 and the soft tissue 65. The composite implant including grip members in an inactive state because the compressible foam in the first configuration is positioned therein.

As shown in FIG. 15B, the medical staff or surgeon can apply pressure, with their hand or a surgical instrument, to the second face 22 of the mesh 20 to cause the compressible foam 30, on the opposite side of the mesh 20, to press into the wet tissue and transition or compress to the second configuration. In addition, the pressure applied to the second face 22 of the mesh 20 may activate the grip members, causing them to extend from the first face of the mesh, to pass through the outer surface of the compressible foam 30 in the second configuration.

FIGS. 15C and 15D illustrate that the activated grip members of FIG. 15B, alone or in combination with the dissolution of the foam 30 in the second configuration, cause the attachment of the composite implant 10 to the tissue 65.

FIG. 15E shows the composite implant 10 after complete removal from the tissue of FIG. 153D. As shown, a majority of the compressible foam is still secured to the grip members on more than half the implant. The only portion of the composite implant 10 wherein the foam is no longer secured to the grip members represents the same portion of the implant upon which pressure was indirectly applied in contact with the biological tissue to activate the grip members prior to attachment to the tissue.

In some embodiments, the step of applying pressure may further include applying pressure to only a first portion of the compressible foam to transition from the first configuration to the second configuration prior to orienting the composite implant and applying pressure to a second different portion of the compressible foam after orienting the composite implant so the first portion of foam in the second configuration allows the grip members positioned thereon to attach to the tissue to anchor the first portion in place while the rest of the composite implant is adjusted or readjusted for proper orienting.

Once the composite implant is properly oriented and the grip members including the compressible foam in the second configuration are attached to the surrounding tissue, the method of repair may further include: fastening the implantable mesh to tissue around the wound with suitable surgical fasteners, such as sutures, staples, adhesives, tacks, and the like; closing the incision, or both.

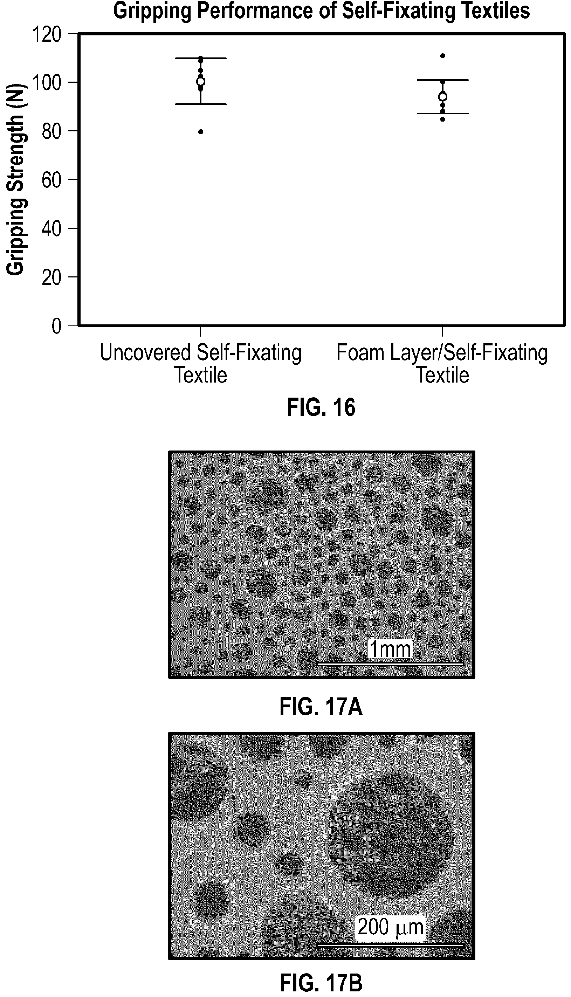

FIG. 16 illustrates that after dissolution of the compressible foam, the gripping performance of the remaining mesh is comparable to the gripping performance of a never-covered self-fixating mesh.

FIGS. 17A and 17B depict the high level of interconnective pores 76 of the compressible foam. FIG. 17A illustrates that due to the high level of interconnective pores 76, any portion of the compressible foam 30 is easily compressed with a finger 75 to transition to the second configuration as shown in FIG. 17B. In both FIGS. 17A and 17B, the foam 30 in the second configuration indirectly exposes the grip members 25 and forms an uneven textured surface.

### EXAMPLE 1

An implantable surgical mesh, having a size of 15x10cm² (rectangular shape) or a size of 12x8cm² (anatomic shape) and including a plurality of grip members extending from a first face of the mesh as described in WO01/81667 was provided. The grip members being produced from a monofilament yarn made of a bioabsorbable material, such as polylactic acid (PLA).

A foam layer, in the form of dry compressible foam, was combined with the grip members of the implantable mesh to form a composite implant, in the following manner:
1) Preparation of the aqueous solution of soluble materials within physiological conditions in order to form a wet foam:
   An aqueous solution containing 5.0 weight percent of hydroxypropyl methylcellulose and 2.5 weight percent of glycerol was prepared as follows.

The hydroxypropyl methylcellulose used had a methoxyl content of 28-30 percent, a hydroxypropyl content of 7-12 percent, and a viscosity of about 50mPa.s⁻¹. The hydroxypropyl methylcellulose used was commercially available from The ShinEtsu Company under the trademark METOLOSE^{®} 60SH. The glycerol used was commercially available from The Merck Company under the Trademark EMPROVE^{®} exp anhydrous glycerol.

First, the hydroxypropyl methylcellulose was dispersed in heated sterile water (80°C) to form an HPMC aqueous dispersion. Glycerol was added to the HPMC aqueous dispersion and cooled at room temperature for 2 hours. The aqueous solution was kept at 4°C until the foaming step. The dynamic viscosity η₀ (zero shear rate) of the aqueous solution at 4°C was about 2Pa.s⁻¹. The dynamic viscosity of the aqueous solution was recorded with DHR2 Rheometer from TA Instruments Company. To determine the dynamic viscosity of the solution, a flow ramp procedure was applied to the solution (0.01Hz initial shear rate to 1000Hz final shear rate) with the help of a 40mm, 2.019° cone steel plate. A Carreau-Yasuda fit was used to determine the zero-rate viscosity value.

### 2) Preparation of a wet non-flow foam material

The obtained aqueous solution in the preceding steps was poured into a mixer bowl. The solution was whisked for 15 minutes at room temperature to introduce air bubbles into the aqueous solution. Following the incorporation of air bubbles, a "non-flow" wet foam material was formed. The wet foam density of the wet foam material was about 0.30g/cm³. Wet foam density was determined from the weight of wet foam required to fill a 185 mL glass container.

### 3) Covering grips of the prothesis fabric

The wet non-flow foam material of Step 2) was casted into ABS frames with a thickness of 1.0mm and over a flat hydrophobic inert support made of Teflon sheet. The wet non-flow foam material was leveled down across the top of the mold using a metallic bar to provide a consistent foam thickness. The frames containing the cast and leveled wet non-flow foam material were then placed into a forced air oven (50°C/5 minutes) to partially solidify or harden the wet foam. The implantable mesh was laid down over the top outer surface of the wet non-flow foam with the grip members facing downward towards the wet foam.

### 4) Drying the wet foam/prothesis fabric

The mesh/wet foam combination was dried in a forced air oven (50°C/2 hours). A composite implant with bioabsorbable grip members, i.e., PLA grip members, at least superficially covered with a layer of a dry compressible foam in a first configuration and having a first thickness was obtained. The foam surface density was equal to 2.1mg/cm². The dried foam represented 22% of the fabric mass.

### EXAMPLE 2

An implantable surgical mesh, having a size of 15x10cm² (rectangular shape) or a size of 12x8cm² (anatomic shape) and including a plurality of grip members extending from a first face of the mesh as described in WO01/81667 was provided. The grip members being produced from a monofilament yarn made of a bioabsorbable material, such as polylactic acid (PLA). The implantable surgical mesh was cleaned with water to remove the residual oil at the surface of yarns.

A foam layer, in the form of dry compressible foam, was combined with the grip members of the implantable mesh to form a composite implant, in the following manner:
1) Preparation of the aqueous solution of soluble materials within physiological conditions in order to form a wet foam:
   An aqueous solution containing 5.0 weight percent of hydroxypropyl methylcellulose and 2.5 weight percent of glycerol was prepared as follows.

The hydroxypropyl methylcellulose used had a methoxyl content of 28-30 percent, a hydroxypropyl content of 7-12 percent, and a viscosity of about 50mPa.s⁻¹. The hydroxypropyl methylcellulose used was commercially available from The ShinEtsu Company under the trademark METOLOSE^{®} 60SH. The glycerol used was commercially available from The Merck Company under the Trademark EMPROVE^{®} exp anhydrous glycerol.

First, the hydroxypropyl methylcellulose was dispersed in heated sterile water (80°C) to form an HPMC aqueous dispersion. Glycerol was added to the HPMC aqueous dispersion and cooled at room temperature for 2 hours. The aqueous solution was kept at 4°C until the foaming step. The dynamic viscosity η₀ (zero shear rate) of the aqueous solution at 4°C was about 2Pa.s⁻¹. The dynamic viscosity of the aqueous solution was recorded with DHR2 Rheometer from TA Instruments Company. To determine the dynamic viscosity of the solution, a flow ramp procedure was applied to the solution (0.01Hz initial shear rate to 1000Hz final shear rate) with the help of a 40mm, 2.019° cone steel plate. A Carreau-Yasuda fit was used to determine the zero-rate viscosity value.

### 2) Preparation of a wet non-flow foam material

The obtained aqueous solution in the preceding steps was poured into a mixer bowl (Kitchen machine MUM 5 from BOSCH company). The solution was whisked for 15 minutes at room temperature to introduce air bubbles into the aqueous solution. Following the incorporation of air bubbles, a "non-flow" wet foam material was formed. The wet foam density of the wet foam material was about 0.30g/cm³. Wet foam density was determined from the weight of wet foam required to fill a 185 mL glass container.

### 3) Covering grips of the prothesis fabric

The wet non-flow foam material of Step 2) was casted into metallic frames with a thickness of 1.5mm and over a flat hydrophobic inert support made of Teflon sheet. The wet non-flow foam material was leveled down across the top of the mold using a metallic bar to provide a consistent foam thickness. The frames containing the cast and leveled wet non-flow foam material were then placed into a forced air oven (50°C/5 minutes) to partially solidify or harden the wet foam. The implantable mesh was laid down over the top outer surface of the wet non-flow foam with the grip members facing downward towards the wet foam.

### 4) Drying the wet foam/prothesis fabric

The mesh/wet foam combination was dried in a forced air oven (50°C/2 hours). A composite implant with bioabsorbable grip members, i.e., PLA grip members, fully covered with a layer of a dry compressible foam in a first configuration and having a first thickness was obtained. The foam surface density was equal to 3.0mg/cm². The dried foam represented 28% of the fabric mass.

### 5) Sterilization of the composite implant

### Composite implants were sterilized with an EtO cycle (38°C for 5 hours, Delta P= 400 mBar)

A gripping strength test was performed comparing: 1) an uncovered self-fixating mesh (GT9); 2) a first composite implant as described herein including a self-fixating mesh including grip members superficially covered with a compressible foam (GT9/HPMC foam of Example 1); and, 3) a second composite implant as described herein including a self-fixating mesh including grip members fully covered with a compressible foam (GT9/HPMC foam of Example 2). Each of the compressible foams including hydroxypropyl methylcellulose (HPMC).

The device used to perform the test is a uniaxial testing machine Instron 5965 equipped with a 50N load cell.

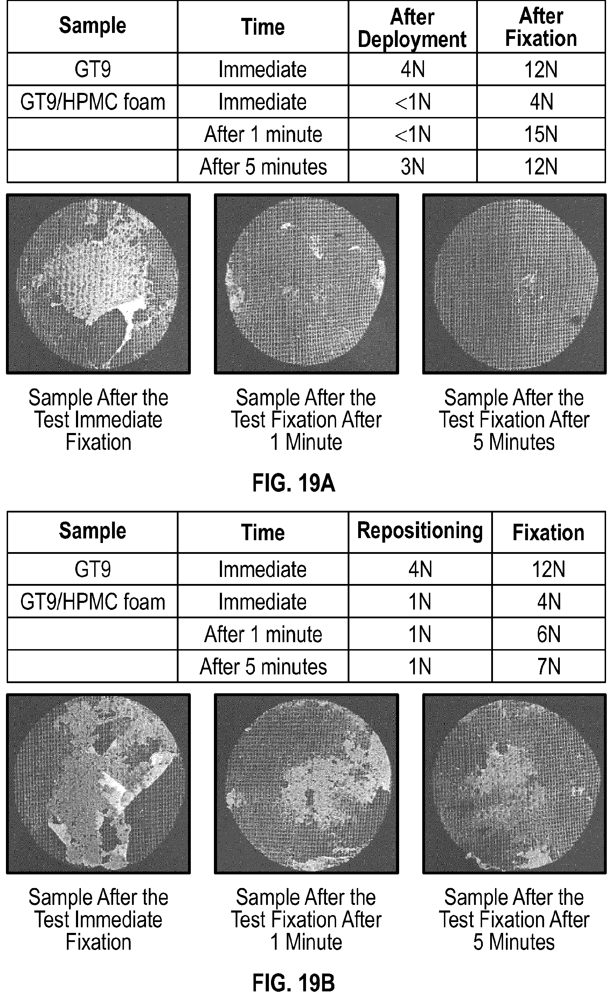

FIG. 19A compares the force required to remove the GT9 uncovered mesh and the first composite implant superficially covered with an HPMC foam of Example 1. The removal force for each was first measured relating to deployment, i.e., measured immediately after deployment, 1 minute after deployment and 5 minutes after deployment. Deployment was simulated by applying a low compression force to a hydrated composite implant. The low compression force was intended to simulate positing the implant at the surgical site without intentionally applying a force to initiate fixation of the implant. As indicated, after deployment, the first composite implant of Example 1 required a smaller removal force, as compared to the uncovered GT9 mesh, because the HPMC foam prevented the grip members from attaching to tissue.

The removal force for both the GT9 uncovered mesh and the first composite implant of Example 1 superficially covered with an HPMC foam were also measured relating to fixation, i.e., immediately after fixation, 1 minute after fixation, and 5 minutes after fixation. Fixation was simulated by applying a high compression force to a hydrated composite implant. The high compression force was intended to simulate fixating the implant to tissue. As indicated, the first composite implant of Example 1 initially required less force than the GT9 uncovered mesh to be removed after fixation. But overtime, the first composite implant of Example 1 required the same, if not more, force to be removed after fixation.

The combination of requiring a smaller initial force for removal following deployment and requiring a force equal to or higher than the GT9 uncovered mesh after fixation makes it easier for the medical staff or surgeon to insert and then possibly reposition the first composite implant of Example 1 after deployment without weakening the full strength of the first composite implant of Example 1 upon fixation.

As further depicted, a majority, i.e., greater than 50%, of the compressible foam was dissolved within 5 minutes of fixation.

**In** some embodiments, the composite implants described herein may be configured to require a removal force of less than 4N after deployment and/or greater than 4N of removal force after self-fixation.

**In** some embodiments, the composite implants described herein may be configured to require a removal force of less than 4N within 5 minutes of deployment and/or greater than 4N of removal force within 5 minutes of self-fixation.

**In** some embodiments, the composite implants described herein may be configured to require a removal force of less than 3N after deployment and/or greater than 6N of removal force after self-fixation.

**In** some embodiments, the composite implants described herein may be configured to require a removal force of less than 3N within 5 minutes of deployment and/or greater than 6N of removal force within 5 minutes of self-fixation.

FIG. 19B compares the force required to remove the GT9 uncovered mesh and the second composite implant of Example 2 fully covered with an HPMC foam. The removal force for each was first measured relating to repositioning, i.e., measured immediately after repositioning, 1 minute after repositioning and 5 minutes after repositioning. As indicated, after repositioning, the second composite implant of Example 2 required, and maintained for at least 5 minutes, a smaller removal force, as compared to the uncovered GT9 mesh, because the HPMC foam prevented the grip members from attaching to tissue.

The removal force for both the GT9 uncovered mesh and the second composite implant of Example 2 fully covered with an HPMC foam were also measured relating to fixation, i.e., immediately after fixation, 1 minute after fixation, and 5 minutes after fixation. As indicated, the second composite implant of Example 2 required less force than the GT9 uncovered mesh to be removed for at least 5 minutes after fixation. It is envisioned that overtime, the second composite implant eventually requires the same, if not more, force to be removed after fixation than the GT9 uncovered mesh.

The combination of requiring and/or maintaining a smaller removal force during repositioning, as well as within 5 minutes after fixation, maximizes the time for the medical staff or surgeon to reposition the second composite implant without weakening the full strength of the second composite long term after fixation.

As depicted, a majority, i.e., greater than 50%, of the compressible foam was dissolved within 5 minutes of fixation.

As further depicted, the composite implants provide about 5 minutes for deployment and/or repositioning of the implant before the implant begins to grip to tissue similar to the uncovered self-fixating mesh.

### EXAMPLE 3

An implantable surgical mesh, having a size of 15x10cm² (rectangular shape) or a size of 12x8cm² (anatomic shape) and including a plurality of grip members extending from a first face of the mesh as described in WO01/81667 was provided. The grip members being produced from a monofilament yarn made of a bioabsorbable material, such as polylactic acid (PLA). The implantable surgical mesh was cleaned with water to remove the residual oil at the surface of yarns.

A foam layer, in the form of dry compressible foam, was combined with the grip members of the implantable mesh to form a composite implant, in the following manner:
1) Preparation of the aqueous solution of soluble materials within physiological conditions in order to form a wet foam:
   An aqueous solution containing 2.5 weight percent of hydroxypropyl methylcellulose, 2.0 sodium hyaluronate and 2.0 weight percent of glycerol was prepared as follows.

The hydroxypropyl methylcellulose used had a methoxyl content of 28-30 percent, a hydroxypropyl content of 7-12 percent, and a viscosity of about 3mPa.s⁻¹. The hydroxypropyl methylcellulose used was commercially available from The ShinEtsu Company under the trademark PHARMACOAT^{®} 603. The glycerol used was commercially available from The Merck Company under the Trademark EMPROVE^{®} exp anhydrous glycerol. The sodium hyaluronate used had an intrinsic viscosity of 1.02m³/Kg. The sodium hyaluronate used was provided by the HTL Biotechnology company.

First, the hydroxypropyl methylcellulose was dispersed in heated sterile water (80°C) to form an HPMC aqueous dispersion. Glycerol was added to the HPMC aqueous dispersion and cooled at room temperature for 2 hours. Then, the sodium hyaluronate was added to the HPMC-Glycerol solution and the solution was mixed 16 hours at room temperature. The dynamic viscosity η₀ (zero shear rate) of the aqueous solution at 20°C was about 9Pa.s⁻¹. The dynamic viscosity of the aqueous solution was recorded with DHR2 Rheometer from TA Instruments Company. To determine the dynamic viscosity of the solution, a flow ramp procedure was applied to the solution (0.01Hz initial shear rate to 1000Hz final shear rate) with the help of a 40mm, 2.019° cone steel plate. A Carreau-Yasuda fit was used to determine the zero-rate viscosity value.

### 2) Preparation of a wet non-flow foam material

The obtained aqueous solution in the preceding steps was poured into a mixer bowl (Kitchen machine Cooking Chef from Kenwood company). The solution was whisked for 2 minutes at room temperature to introduce air bubbles into the aqueous solution. Following the incorporation of air bubbles, a "non-flow" wet foam material was formed. The wet foam density of the wet foam material was about 0.26g/cm³. Wet foam density was determined from the weight of wet foam required to fill a 185 mL glass container.

### 3) Covering grips of the prothesis fabric

The wet non-flow foam material of Step 2) was casted into metallic frames with a thickness of 1.5mm and over a flat hydrophobic inert support made of Teflon sheet. The wet non-flow foam material was leveled down across the top of the mold using a metallic bar to provide a consistent foam thickness. The implantable mesh was laid down over the top outer surface of the wet non-flow foam with the grip members facing downward towards the wet foam.

### 4) Drying the wet foam/prothesis fabric

The frames containing the implantable mesh and the wet non-flow foam material were then placed into an oven (28°C/35% relative humidity). The mesh/wet foam combination was dried 16 hours (28°C/35% relative humidity). A composite implant with bioabsorbable grip members, i.e., PLA grip members, at least superficially covered with a layer of a dry compressible foam in a first configuration and having a first thickness was obtained. The foam surface density was equal to 3.5mg/cm². The dried foam represented 33% of the fabric mass.

### 5) Sterilization of the composite implant

Composite implants were sterilized with an EtO cycle (38°C for 5 hours, Delta P= 400 mBar).

### EXAMPLE 4

An implantable surgical mesh, having a size of 15x10cm² (rectangular shape) or a size of 12x8cm² (anatomic shape) and including a plurality of grip members extending from a first face of the mesh as described in WO01/81667 was provided. The grip members being produced from a monofilament yarn made of a bioabsorbable material, such as polylactic acid (PLA). The implantable surgical mesh was cleaned with water to remove the residual oil at the surface of yarns.

A foam layer, in the form of dry compressible foam, was combined with the grip members of the implantable mesh to form a composite implant, in the following manner:
1) Preparation of the aqueous solution of soluble materials within physiological conditions in order to form a wet foam:
   An aqueous solution containing 2.5 weight percent of collagen, and 0.25 weight percent of glycerol was prepared as follows.

The collagen used had was provided by The Sofradim Production company under the Trademark CPP.

First, the collagen and the glycerol were poured in heated sterile water (30°C). The solution was mixed 4hours at 30°C. The dynamic viscosity η₀ (zero shear rate) of the aqueous solution at 30°C was about 22Pa.s⁻¹. The dynamic viscosity of the aqueous solution was recorded with DHR2 Rheometer from TA Instruments Company. To determine the dynamic viscosity of the solution, a flow ramp procedure was applied to the solution (0.01Hz initial shear rate to 1000Hz final shear rate) with the help of a 40mm, 2.019° cone steel plate. A Carreau-Yasuda fit was used to determine the zero-rate viscosity value.

### 2) Preparation of a wet non-flow foam material

The obtained aqueous solution in the preceding steps was poured into a mixer bowl (Kitchen machine Cooking Chef from Kenwood company). The solution was whisked for 2 minutes at 30°C to introduce air bubbles into the aqueous solution. Following the incorporation of air bubbles, a "non-flow" wet foam material was formed. The wet foam density of the wet foam material was about 0.23g/cm³. Wet foam density was determined from the weight of wet foam required to fill a 185 mL glass container.

### 3) Covering grips of the prothesis fabric

The wet non-flow foam material of Step 2) was casted into metallic frames with a thickness of 1.5mm and over a flat hydrophobic inert support made of Teflon sheet. The wet non-flow foam material was leveled down across the top of the mold using a metallic bar to provide a consistent foam thickness. The implantable mesh was laid down over the top outer surface of the wet non-flow foam with the grip members facing downward towards the wet foam.

### 4) Drying the wet foam/prothesis fabric

The frames containing the implantable mesh and the wet non-flow foam material were then placed into an oven (20°C/40% relative humidity). The mesh/wet foam combination was dried 16 hours (20°C/40% relative humidity). A composite implant with bioabsorbable grip members, i.e., PLA grip members, at least superficially covered with a layer of a dry compressible foam in a first configuration and having a first thickness was obtained. The foam surface density was equal to 1.3mg/cm². The dried foam represented 14% of the fabric mass.

### 5) Sterilization of the composite implant

The composite implant was not sterilized.

### EXAMPLE 5

An implantable surgical mesh, having a size of 15x10cm² (rectangular shape) or a size of 12x8cm² (anatomic shape) and including a plurality of grip members extending from a first face of the mesh as described in WO01/81667 was provided. The grip members being produced from a monofilament yarn made of a bioabsorbable material, such as polylactic acid (PLA). The implantable surgical mesh was cleaned with water to remove the residual oil at the surface of yarns.

A foam layer, in the form of dry compressible foam, was combined with the grip members of the implantable mesh to form a composite implant, in the following manner:
1) Preparation of the aqueous solution of soluble materials within physiological conditions in order to form a wet foam:
   An aqueous solution containing 2.5 weight percent of collagen, and 0.25 weight percent of glycerol was prepared as follows.

The collagen used had was provided by The Sofradim Production company under the Trademark CPP.

First, the collagen and the glycerol were poured in heated sterile water (30°C). The solution was mixed 4hours at 30°C. The dynamic viscosity η₀ (zero shear rate) of the aqueous solution at 30°C was about 22Pa.s⁻¹. The dynamic viscosity of the aqueous solution was recorded with DHR2 Rheometer from TA Instruments Company. To determine the dynamic viscosity of the solution, a flow ramp procedure was applied to the solution (0.01Hz initial shear rate to 1000Hz final shear rate) with the help of a 40mm, 2.019° cone steel plate. A Carreau-Yasuda fit was used to determine the zero-rate viscosity value.

### 2) Preparation of a wet non-flow foam material

The obtained aqueous solution in the preceding steps was poured into a mixer bowl (Kitchen machine Cooking Chef from Kenwood company). The solution was whisked for 2 minutes at 30°C to introduce air bubbles into the aqueous solution. Following the incorporation of air bubbles, a "non-flow" wet foam material was formed. The wet foam density of the wet foam material was about 0.23g/cm³. Wet foam density was determined from the weight of wet foam required to fill a 185 mL glass container.

### 3) Covering grips of the prothesis fabric

The wet non-flow foam material of Step 2) was casted into metallic frames with a thickness of 1.5mm and over a flat hydrophobic inert support made of Teflon sheet. The wet non-flow foam material was leveled down across the top of the mold using a metallic bar to provide a consistent foam thickness. The implantable mesh was laid down over the top outer surface of the wet non-flow foam with the grip members facing downward towards the wet foam.

### 4) Drying the wet foam/prothesis fabric

The frames containing the implantable mesh and the wet non-flow foam material were then placed into an oven (20°C/40% relative humidity). The mesh/wet foam combination was dried 16 hours (20°C/40% relative humidity). A composite implant with bioabsorbable grip members, i.e., PLA grip members, at least superficially covered with a layer of a dry compressible foam in a first configuration and having a first thickness was obtained. The foam surface density was equal to 1.3mg/cm². The dried foam represented 14% of the fabric mass. The foam was not sterilized.

### 5) Sterilization of the composite implant

Composite implants were sterilized with an EtO cycle (38°C for 5 hours, Delta P= 400 mBar).

### EXAMPLE 6

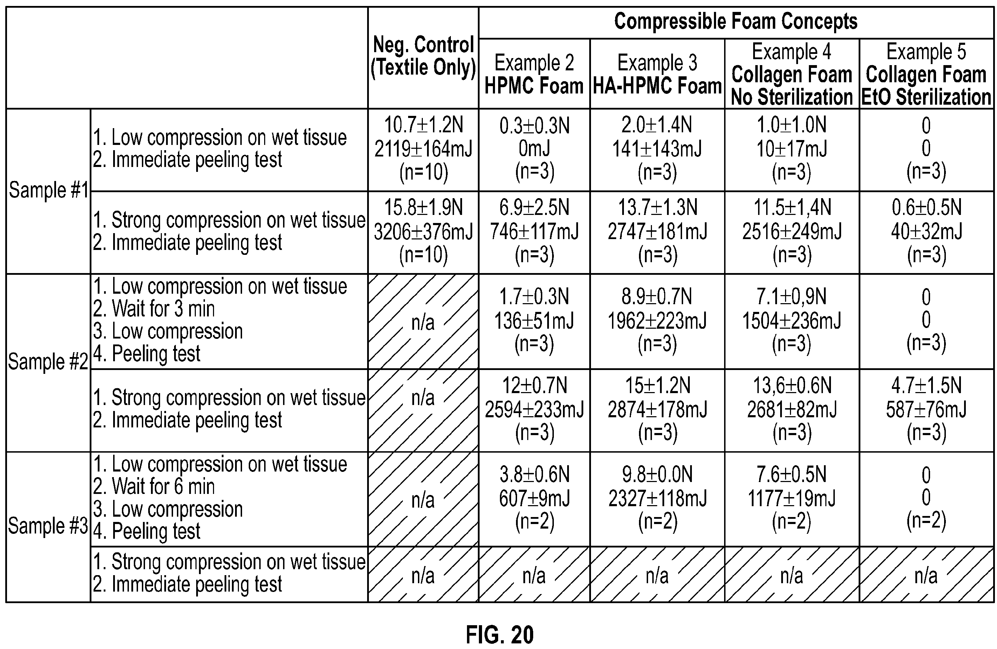

Methods were performed to assess foam efficiency to inactivate and/or activate the self-gripping ability of the composite implants described herein. Specifically, multiple samples of each of the composite implants as provided in Examples 2-5 were individually exposed to either an inactivation gripping test alone or an inactivation gripping test followed by an activation gripping test within 15 minutes of the inactivation test to assess foam efficiency. The results of both tests are shown in FIG. 20.

The inactivation gripping test was performed, under low compression, to determine the inactive gripping ability of each of the samples of composite implants as provided in Examples 2-5. Low compression was used to generally mimic the amount of pressure associated with deploying and/or properly orienting a composite implant in the tissue of a patient. Various time lengths were allowed to pass before evaluating the gripping performance under low compression.

The activation gripping test was performed, under high compression, to determine the active gripping ability of each of the samples of composite implants as provided in Examples 2-5. High compression was used to generally mimic the amount of pressure associated with fixing the composite implant to the tissue of a patient after deployment and/or proper orientation. The implant self-fixated to the tissue via the active grip members during and/or after high compression.

An abrasion tester (Elcometer ^{®} 1720 Abrasion Tester) was used to apply compression, e.g., low compression or high compression, across each of the samples and an Instron^{®} universal testing machine was used to separate, i.e., ungrip, each sample from a wetted porous substrate designed to simulated wet tissue.

### Inactivation Gripping Test:

A porous substrate (16x12cm), such as a porous filter, was affixed to and centered on a larger metal plate (30x12cm). The porous substrate having a 5mm thickness.

The porous substrate, including at least a portion of the metal plate affixed thereto, was wetted by placing it in a bath of saline solution at 37°C for a period of time. Upon removal from the bath, the wet substrate (and the plate) was made to stream excess saline solution from the pores of the substrate back to the bath by holding the substrate generally vertically over the bath for a brief period of time.

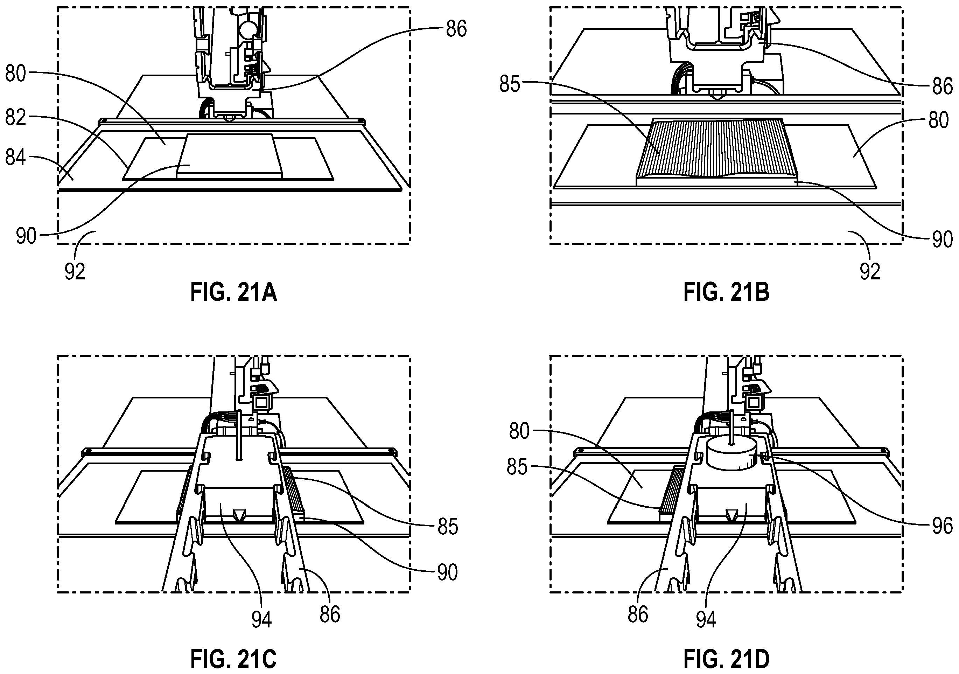

As shown in FIG. 21A, the metal plate 80, with the wet porous substrate 90 affixed thereto, was positioned within an opening 82 (30x12cm) defined within a planar metal support 84 (48x17cm) secured to a top surface of an abrasion tester 92 (Elcometer ^{®} 1720 Abrasion Tester). The nesting of the metal plate 80 within the opening 82 defined within the metal support 84 secured to the top surface of the tester 92 ensures the repeatable positioning of the porous substrate 90 on the tester 92.

As shown in FIGS. 21B-21C, a sample composite implant 85 (of any of Examples 2-5) was positioned on a top surface of the wet porous substrate 90 prior to lowering a carriage 86 from a vertical position to a horizontal position over the implant 85. A compression device 94 (without an additional weight) was added to the carriage 86 and the carriage 86 including the compression device 94 was slid laterally across the implant 85 to apply low compression thereto. The compression device 94 weighed about 146g. The carriage 86 was set at a speed of 10 and set to perform two cycles on the tester 92.

At the end of the lateral movement of the carriage 86, the compression device 94 was removed from the carriage 86 and the carriage 86 was returned to the vertical position.

A first group of samples of each of the Examples 2-5 were tested immediately (T0) following low compression. This group of samples were immediately taken from the abrasion tester, without touching the samples to avoid applying any additional compression to the samples, and secured in a lower jaw member of an Instron^{®} universal testing machine.

A second and third group of samples of each of the Examples 2-5 were tested after a longer hydration time (T3minutes or T6minutes, respectively). A timer was set after application of the initial low compression for each of the second and third groups. At the end of the extended hydration time, a low compression as described hereinabove was reapplied to each of the samples prior to being removed from the abrasion tester and secured in a lower jaw member of an Instron^{®} universal testing machine.

In the Instron^{®} testing machine, a pair of clasps were attached to a lower extremity of the sample to grasp at least two pore pillars. The clasps were connected to a load cell at the top of the testing machine opposite the lower jaw member via a polyamide yarn. The load cell applied an increasing amount of force (N) and work (mJ) through the yarn to the clasps until the sample was separated, i.e., ungripped, from the porous substrate.

### Activation Gripping Test:

A porous substrate (16x12cm), such as a porous filter, was affixed to and centered on a larger metal plate (30x12cm). The porous substrate having a 5mm thickness.

The porous substrate, including at least a portion of the metal plate affixed thereto, was wetted by placing it in a bath of saline solution at 37°C for a period of time. Upon removal from the bath, the wet substrate (and the plate) was made to stream excess saline solution from the pores of the substrate back to the bath by holding the substrate generally vertically over the bath for a brief period of time.

The metal plate, with the wet porous substrate affixed thereto, was positioned within an opening (30x12cm) defined within a planar metal support (48x17cm) secured to a top surface of an abrasion tester (Elcometer ^{®} 1720 Abrasion Tester). The nesting of the metal plate within the opening defined within the metal support secured to the top surface of the tester ensures the repeatable positioning of the porous substrate on the tester.

A sample composite implant (of any of Examples 2-5) was positioned on a top surface of the wet porous substrate prior to lowering a carriage from a vertical position to a horizontal position over the implant. A compression device (without an additional weight) was added to the carriage and the carriage including the compression device was slid laterally across the implant to provide low compression thereto. The compression device weighed about 146g. The carriage 86 was set at a speed of 10 and set to perform two cycles on the tester 92.

A first group of samples of each of the Examples 2-5 were tested immediately (T0) following low compression. This group of samples were immediately taken from the abrasion tester, without touching the samples to avoid applying any additional compression to the samples, and secured in a lower jaw member of an Instron^{®} universal testing machine.

A second group of samples of each of the Examples 2-5 were tested after a longer hydration time (T3minutes). A timer was set after low compression for the second group. At the end of the extended hydration time, the samples were taken from the abrasion tester, without touching the samples to avoid applying any additional compression to the samples, and secured in a lower jaw member of an Instron^{®} universal testing machine.

**In** the Instron^{®} testing machine, a pair of clasps were attached to a lower extremity of the sample to grasp at least two pore pillars. The clasps were connected to a load cell at the top of the testing machine opposite the lower jaw member via a polyamide yarn. The load cell applied an increasing amount of force (N) and work (mJ) through the yarn to the clasps until the sample was separated, i.e., ungripped, from the porous substrate.

After the inactivation gripping test, the porous substrate, including at least a portion of the metal plate affixed thereto, was wetted by placing it in a bath of saline solution at 37°C for a period of time. Upon removal from the bath, the wet substrate (and the plate) was made to stream excess saline solution from the pores of the substrate back to the bath by holding the substrate generally vertically over the bath for a brief period of time.

The metal plate, with the wet porous substrate affixed thereto, was positioned within an opening (30x12cm) defined within a planar metal support (48x17cm) secured to a top surface of an abrasion tester (Elcometer ^{®} 1720 Abrasion Tester). The nesting of the metal plate within the opening defined within the metal support secured to the top surface of the tester ensures the repeatable positioning of the porous substrate on the tester.

The sample composite was positioned on a top surface of the wet porous substrate prior to lowering a carriage from a vertical position to a horizontal position over the implant. A compression device (without an additional weight) was added to the carriage. A weight 96 of 300g was added to the compression device and the carriage including the weighted compression device was slid laterally across the implant a second time to provide high compression thereto (FIG. 21D).

At the end of the lateral movement of the carriage, the weighted compression device was removed from the carriage and the carriage was returned to the vertical position. The samples were taken from the abrasion tester, without touching the samples to avoid applying any additional compression to the samples, and secured in a lower jaw member of an Instron^{®} universal testing machine.

In the Instron^{®} testing machine, a pair of clasps were attached to a lower extremity of the sample to grasp at least two pore pillars. The clasps were connected to a load cell at the top of the testing machine opposite the lower jaw member via a polyamide yarn. The load cell applied an increasing amount of force (N) and work (mJ) through the yarn to the clasps until the sample was separated, i.e., ungripped, from the porous substrate.

As the results demonstrate in FIG. 20, in some embodiments, the composite implants described herein may be configured to transition from inactive to active gripping configurations in 6 minutes or less, and particularly 3 minutes or less, of an application of low and/or high compression thereto.

In some embodiments, the composite implants of Examples 3 and 4 (HA-HPMA foam and Collagen Foam not sterilized, respectively) may be better suited than the composite implants of Example 5 (EtO sterilized Collagen) for use in open surgical procedures due to the faster transition times from inactive to active gripping configurations.

Alternatively, in some embodiments, the composite implants of Example 5 (EtO sterilized Collagen) may be better suited than the composite implants of Examples 3 and 4 (HA-HPMA foam and Collagen Foam not sterilized, respectively) for use in minimally invasive procedures which may require longer deployment/fixation times than open surgical procedures.

## Claims

1. A composite implant (10) comprising:
an implantable mesh (20) comprising a mesh body (23) including a first face (21) and a second face (22) opposite the first face,
a plurality of grip members (25) extending from the first face (21) of the mesh (20), each of the plurality of grip members (25) include a body extending between a first end attached to the mesh and a second end free of the mesh (20),
**characterized in that** it further comprises:
a compressible foam (30) covering at least the second ends of the plurality of grip members (25), the compressible foam (30) configured to transition from a first foam configuration having a first thickness to a second foam configuration having a second thickness smaller than the first thickness upon the application of pressure thereto,
wherein the grip members (25) are configured to transition from a first inactive gripping configuration covered by the foam (30) to a second active gripping configuration upon the removal of the foam for fixating the grip members (25) and/or implant (10) to the tissue.

2. The composite implant (10) of claim 1, wherein grip members (25) are concealed within the compressible foam (30) and in an inactive gripping configuration in the first foam configuration and indirectly exposed by the compressible foam and in an inactive configuration in the second foam configuration, wherein in its second configuration, the foam is compressed and follows the outer contour of the head portions of the grip members (25) thereby displaying an uneven texture indicative of the general location of grip members beneath the compressed foam; and/or
the grip members (25) are configured to transition from the inactive gripping configuration to an active gripping configuration upon dissolving of the compressible foam.

3. The composite implant (10) of claim 1, wherein:
(a) the compressible foam (30) in the first configuration further covers the body (26) of each of the plurality of grip members (25); or
(b) the first thickness of the foam (30) is greater than or equal to a length of the body (26) of each of the plurality of grip members (25); or
(c) the compressible foam (30) in the first configuration further covers only a portion of the body (26) of each of the plurality of grip members (25); or
(d) the first thickness of the foam (30) is smaller than a length of each of the plurality of grip members (25).

4. The composite implant (10) of claim 1, wherein the composite implant (10) further includes a gap (40) between the first face (21) of the mesh (20) and a second surface of the compressible foam (30) in the first configuration and optionally the second configuration.

5. The composite implant (10) of claim 1, wherein the mesh (20) further includes at least one of a slit (29), a central opening (28), or both and the compressible foam (30) is positioned along at least one of the slit (29), the opening (28), or both.

6. The composite implant (10) of claim 1, wherein the plurality of grip members (25) are concealed within the compressible foam (30) and in an inactive gripping configuration in both the first and second foam configurations; or
the grip members (25) are concealed within the compressible foam (30) and in an inactive gripping configuration in the first foam configuration and are directly exposed in an active gripping configuration in the second foam configuration.

7. A composite implant (10) according to claim 5, further comprising a flap mesh (35) including a first flap face and a second flap face opposite the first flap face, the flap mesh (35) extending across the slit (29) and including a first portion of the second flap face overlapping a first portion of the first mesh face (21), wherein the plurality of grip members (25) extends from the first portion of the first mesh face (21), each of the plurality of grip members (25) including a body (26) extending between a first attached end and a second free end.

8. A method of manufacturing a composite implant (10) according to any of claims 1-7, comprising:
combining a biocompatible polymeric foaming agent, a biocompatible plasticizer, and a solvent to form a solution,
combining the solution with a gas to form a wet non-flow foam material,
applying the wet non-flow foam material to at least a second free end of each of a plurality of grip members (25) extending from a first face (21) of an implantable mesh (20),
drying the wet non-flow foam to form the composite implant (10) including a compressible foam (30) in a first configuration having a first thickness, the compressible foam (30) configured to transition from the first foam configuration having the first thickness to a second foam configuration having a second thickness smaller than the first thickness,
wherein the grip members (25) are configured to transition from a first inactive gripping configuration covered by the foam (30) to a second active gripping configuration upon the removal of the foam for fixating the grip members (25) and/or implant (10) to the tissue.

9. The method of manufacturing a composite implant (10) of claim 8, wherein the biocompatible polymeric foaming agent is selected from the group consisting gelatins, collagens, poloxamers, polysorbates, methylcellulose (MC), hydroxypropyl methylcellulose (HPMC), hydroxypropyl cellulose (HPC), hydroxyethyl cellulose (HEC) and mixtures thereof; and, optionally, wherein:
(a) the biocompatible polymeric foaming agent is hydroxypropyl methylcellulose, and optionally
(i) the hydroxypropyl methylcellulose includes a methyl substitution degree from 1 to 2.5; and/or
(b) the biocompatible polymeric foaming agent represents a concentration ranging from about 0.1 to about 20 weight percent of the aqueous solution.

10. The method of manufacturing a composite implant (10) of claim 8, wherein the biocompatible plasticizer is selected from the group consisting glycerol, sorbitol, xylitol, mannitol, propylene glycol, polyethylene glycol, and mixtures thereof; and, optionally, wherein:
(a) the biocompatible polymeric plasticizer is at least one of glycerol, sorbitol, or both; or
(b) the biocompatible plasticizer represents a concentration ranging from about 0.5 to about 40 weight percent of the aqueous solution.

11. The method of manufacturing a composite implant (10) of claim 8, wherein the solvent is selected from the group consisting of spring water, sterile water, distilled water, deionized water, and combinations thereof.

12. The method of manufacturing a composite implant (10) of claim 8, wherein the aqueous solution further comprises a biocompatible additive selected from the group consisting of alginate, pectin, carrageenan, methylcellulose (MC), hydroxypropyl methylcellulose (HPMC), hydroxypropyl cellulose (HPC), hydroxyethyl cellulose (HEC), xanthan gum, guar gum, dextran, hyaluronan, pullulan, maltodextrin, gelatins, collagens, polyvinyl-alcohol, polyethylene-glycol and mixtures thereof; and, optionally, wherein:
(a) the biocompatible additive is hydroxypropyl methylcellulose with a methyl substitution degree from 1 to 2.5 and a hydroxypropyl molar substitution from 0.1 to 1; or
(b) the biocompatible additive represents a concentration ranging from about 0.1 to about 10 weight percent of the aqueous solution.

13. The method of manufacturing a composite implant (10) of claim 8, wherein combining the solution with a gas to form a wet non-flow foam material comprises whisking the solution to incorporate air bubbles into the solution to form the wet non-flow foam material.

14. The method of manufacturing a composite implant (10) of claim 8, wherein applying the wet non-flow foam material to at least a second free end of each of a plurality of grip members (25) extending from a first face of an implantable mesh (20) comprises casting the wet non-flow foam material over an inert support to a thickness sufficient to cover at least the free end of each plurality of grip members (25) and placing the implantable mesh (20) with the grip-members (25) facing towards the wet non-flow material over a first top surface of the wet non-flow material.

15. The method of manufacturing a composite implant (10) of claim 8, wherein drying the wet non-flow foam material comprises heating the wet non-flow foam material in an oven to about 50° C.

## Patentansprüche

1. Zusammengesetztes Implantat (10), umfassend:
ein implantierbares Netz (20), das einen Netzkörper (23) umfasst, der eine erste Fläche (21) und eine zweite Fläche (22) gegenüber der ersten Fläche aufweist,
mehrere Griffelemente (25), die sich von der ersten Fläche (21) des Netzes (20) erstrecken, wobei jedes der mehreren Griffelemente (25) einen Körper aufweist, der sich zwischen einem am Netz angebrachten ersten Ende und einem zweiten Ende, das frei vom Netz (20) ist, erstreckt,
**dadurch gekennzeichnet, dass** es ferner Folgendes umfasst:
einen komprimierbaren Schaumstoff (30), der mindestens die zweiten Enden der mehreren Griffelemente (25) bedeckt, wobei der komprimierbare Schaumstoff (30) dazu ausgelegt ist, beim Aufbringen von Druck darauf von einer ersten Schaumstoffkonfiguration mit einer ersten Dicke zu einer zweiten Schaumstoffkonfiguration mit einer zweiten Dicke, die kleiner als die erste Dicke ist, überzugehen,
wobei die Griffelemente (25) dazu ausgelegt sind, nach dem Entfernen des Schaumstoffs zum Fixieren der Griffelemente (25) und/oder des Implantats (10) an dem Gewebe von einer ersten inaktiven Griffkonfiguration, die durch den Schaumstoff (30) bedeckt ist, in eine zweite aktive Griffkonfiguration überzugehen.

2. Zusammengesetztes Implantat (10) nach Anspruch 1, wobei Griffelemente (25) in der ersten Schaumstoffkonfiguration innerhalb des komprimierbaren Schaumstoffs (30) verborgen und in einer inaktiven Griffkonfiguration sind und in der zweiten Schaumstoffkonfiguration durch den komprimierbaren Schaumstoff indirekt freigelegt und in einer inaktiven Konfiguration sind, wobei der Schaumstoff in der zweiten Konfiguration komprimiert wird und der Außenkontur der Kopfabschnitte der Griffelemente (25) folgt, wodurch eine ungleichmäßige Struktur offenbart wird, die die allgemeine Lage der Griffelemente unter dem komprimierten Schaumstoff anzeigt; und/oder
wobei die Griffelemente (25) dazu ausgelegt sind, beim Auflösen des komprimierbaren Schaumstoffs von der inaktiven Griffkonfiguration in eine aktive Griffkonfiguration überzugehen.

3. Zusammengesetztes Implantat (10) nach Anspruch 1, wobei:
(a) der komprimierbare Schaumstoff (30) in der ersten Konfiguration ferner den Körper (26) jedes der mehreren Griffelemente (25) bedeckt oder
(b) die erste Dicke des Schaumstoffs (30) größer oder gleich einer Länge des Körpers (26) jedes der mehreren Griffelemente (25) ist oder
(c) der komprimierbare Schaumstoff (30) in der ersten Konfiguration ferner nur einen Abschnitt des Körpers (26) jedes der mehreren Griffelemente (25) bedeckt oder
(d) die erste Dicke des Schaumstoffs (30) kleiner als eine Länge jedes der mehreren Griffelemente (25) ist.

4. Zusammengesetztes Implantat (10) nach Anspruch 1, wobei das zusammengesetzte Implantat (10) ferner einen Spalt (40) zwischen der ersten Fläche (21) des Netzes (20) und einer zweiten Fläche des komprimierbaren Schaumstoffs (30) in der ersten Konfiguration und optional der zweiten Konfiguration aufweist.

5. Zusammengesetztes Implantat (10) nach Anspruch 1, wobei das Netz (20) ferner einen Schlitz (29) und/oder eine zentrale Öffnung (28) aufweist und der komprimierbare Schaumstoff (30) entlang des Schlitzes (29) und/oder der Öffnung (28) positioniert ist.

6. Zusammengesetztes Implantat (10) nach Anspruch 1, wobei die mehreren Griffelemente (25) innerhalb des komprimierbaren Schaumstoffs (30) und in einer inaktiven Griffkonfiguration sowohl in der ersten als auch der zweiten Schaumstoffkonfiguration verborgen sind oder die Griffelemente (25) innerhalb des komprimierbaren Schaumstoffs (30) und in einer inaktiven Griffkonfiguration in der ersten Schaumstoffkonfiguration verdeckt sind und in einer aktiven Griffkonfiguration in der zweiten Schaumstoffkonfiguration direkt freigelegt sind.

7. Zusammengesetztes Implantat (10) nach Anspruch 5, ferner umfassend einen Netzlappen (35), der eine erste Lappenfläche und eine zweite Lappenfläche gegenüber der ersten Lappenfläche aufweist, wobei sich der Netzlappen (35) über den Schlitz (29) erstreckt und einen ersten Abschnitt der zweiten Lappenfläche aufweist, der einen ersten Abschnitt der ersten Netzfläche (21) überlagert, wobei sich die mehreren Griffelemente (25) vom ersten Abschnitt der ersten Netzfläche (21) erstrecken, wobei jedes der mehreren Griffelemente (25) einen Körper (26) aufweist, der sich zwischen einem ersten befestigten Ende und einem zweiten freien Ende erstreckt.

8. Verfahren zum Herstellen eines zusammengesetzten Implantats (10) nach einem der Ansprüche 1-7, umfassend:
Kombinieren eines biokompatiblen polymeren Schaumbildners, eines biokompatiblen Weichmachers und eines Lösungsmittels, um eine Lösung auszubilden,
Kombinieren der Lösung mit einem Gas, um ein feuchtes, nicht fließfähiges Schaumstoffmaterial auszubilden,
Auftragen des feuchten, nicht fließfähigen Schaumstoffmaterials auf mindestens ein zweites freies Ende jedes mehrerer Griffelemente (25), die sich von einer ersten Fläche (21) eines implantierbaren Netzes (20) erstrecken,
Trocknen des feuchten, nicht fließfähigen Schaumstoffs, um das zusammengesetzte Implantat (10) auszubilden, das einen komprimierbaren Schaumstoff (30) in einer ersten Konfiguration mit einer ersten Dicke aufweist, wobei der komprimierbare Schaumstoff (30) dazu ausgelegt ist, von der ersten Schaumstoffkonfiguration mit der ersten Dicke in eine zweite Schaumstoffkonfiguration mit einer zweiten Dicke, die kleiner als die erste Dicke ist, überzugehen, wobei die Griffelemente (25) dazu ausgelegt sind, nach dem Entfernen des Schaumstoffs zum Fixieren der Griffelemente (25) und/oder des Implantats (10) an dem Gewebe von einer ersten inaktiven Griffkonfiguration, die durch den Schaumstoff (30) bedeckt ist, in eine zweite aktive Griffkonfiguration überzugehen.

9. Verfahren zur Herstellung eines zusammengesetzten Implantats (10) nach Anspruch 8, wobei der biokompatible polymere Schaumbildner aus der Gruppe bestehend aus Gelatinen, Kollagenen, Poloxameren, Polysorbaten, Methylcellulose (MC), Hydroxypropylmethylcellulose (HPMC), Hydroxypropylcellulose (HPC), Hydroxyethylcellulose (HEC) und Mischungen davon ausgewählt ist und optional wobei:
(a) der biokompatible polymere Schaumbildner Hydroxypropylmethylcellulose ist und optional (i) die Hydroxypropylmethylcellulose einen Methylsubstitutionsgrad von 1 bis 2,5 einschließt und/oder
(b) der biokompatible polymere Schaumbildner eine Konzentration im Bereich von etwa 0,1 bis etwa 20 Gew.-% der wässrigen Lösung darstellt.

10. Verfahren zur Herstellung eines zusammengesetzten Implantats (10) nach Anspruch 8, wobei der biokompatible Weichmacher aus der Gruppe bestehend aus Glycerin, Sorbit, Xylit, Mannitol, Propylenglykol, Polyethylenglykol und Mischungen davon ausgewählt ist und optional wobei:
(a) der biokompatible polymere Weichmacher Glycerin und/oder Sorbit ist oder
(b) der biokompatible Weichmacher eine Konzentration im Bereich von etwa 0,5 bis etwa 40 Gew.-% der wässrigen Lösung darstellt.

11. Verfahren zur Herstellung eines zusammengesetzten Implantats (10) nach Anspruch 8, wobei das Lösungsmittel ausgewählt ist aus der Gruppe bestehend aus Quellwasser, sterilem Wasser, destilliertem Wasser, deionisiertem Wasser und Kombinationen davon.

12. Verfahren zur Herstellung eines zusammengesetzten Implantats (10) nach Anspruch 8, wobei die wässrige Lösung ferner ein biokompatibles Additiv umfasst, das aus der Gruppe bestehend aus Alginat, Pektin, Carrageenan, Methylcellulose (MC), Hydroxypropylmethylcellulose (HPMC), Hydroxypropylcellulose (HPC), Hydroxyethylcellulose (HEC), Xanthan, Guargummi, Dextran, Hyaluronan, Pullulan, Maltodextrin, Gelatinen, Kollagenen, Polyvinylalkohol, Polyethylenglykol und Mischungen davon ausgewählt ist und optional wobei:
(a) das biokompatible Additiv Hydroxypropylmethylcellulose mit einem Methylsubstitutionsgrad von 1 bis 2,5 und einer Hydroxypropylmolsubstitution von 0,1 bis 1 ist oder
(b) das biokompatible Additiv eine Konzentration im Bereich von etwa 0,1 bis etwa 10 Gew.-% der wässrigen Lösung darstellt.

13. Verfahren zur Herstellung eines zusammengesetzten Implantats (10) nach Anspruch 8, wobei das Kombinieren der Lösung mit einem Gas, um ein feuchtes, nicht fließfähiges Schaumstoffmaterial auszubilden, das Aufschlagen der Lösung umfasst, um Luftblasen in die Lösung zu integrieren, um das feuchte, nicht fließfähige Schaumstoffmaterial auszubilden.

14. Verfahren zum Herstellen eines zusammengesetzten Implantats (10) nach Anspruch 8, wobei das Auftragen des feuchten, nicht fließfähigen Schaumstoffmaterials auf mindestens ein zweites freies Ende jedes mehrerer Griffelemente (25), die sich von einer ersten Fläche eines implantierbaren Netzes (20) erstrecken, das Gießen des feuchten, nicht fließfähigen Schaumstoffmaterials über einen inerten Träger bis zu einer Dicke, die ausreicht, um zumindest das freie Ende jedes der mehreren Griffelemente (25) zu bedecken, und das Platzieren des implantierbaren Netzes (20), wobei die Griffelemente (25) dem feuchten, nicht fließfähigen Material zugewandt sind, über einer ersten oberen Fläche des feuchten, nicht fließfähigen Materials umfasst.

15. Verfahren zur Herstellung eines zusammengesetzten Implantats (10) nach Anspruch 8, wobei das Trocknen des feuchten, nicht fließfähigen Schaumstoffmaterials das Erwärmen des feuchten, nicht fließfähigen Schaumstoffmaterials in einem Ofen auf etwa 50 °C umfasst.

## Revendications

1. Implant composite (10) comprenant :
une maille implantable (20) comprenant un corps de maille (23) comportant une première face (21) et une seconde face (22) opposée à la première face,
une pluralité d'éléments de préhension (25) s'étendant à partir de la première face (21) de la maille (20), chacun de la pluralité d'éléments de préhension (25) comprenant un corps s'étendant entre une première extrémité fixée à la maille et une seconde extrémité séparée de la maille (20),
**caractérisé en ce qu'**il comprend en outre :
une mousse compressible (30) recouvrant au moins les secondes extrémités de la pluralité d'éléments de préhension (25), la mousse compressible (30) étant conçue pour passer d'une première configuration de mousse ayant une première épaisseur à une seconde configuration de mousse ayant une seconde épaisseur inférieure à la première épaisseur lors de l'application d'une pression sur celle-ci,
les éléments de préhension (25) étant conçus pour passer d'une première configuration de préhension inactive recouverte par la mousse (30) à une seconde configuration de préhension active lors du retrait de la mousse pour fixer les éléments de préhension (25) et/ou l'implant (10) au tissu.

2. Implant composite (10) selon la revendication 1, les éléments de préhension (25) étant dissimulés à l'intérieur de la mousse compressible (30) et dans une configuration de préhension inactive dans la première configuration de mousse et indirectement exposés par la mousse compressible et dans une configuration inactive dans la seconde configuration de mousse, dans sa seconde configuration, la mousse étant comprimée et suivant le contour extérieur des parties tête des éléments de préhension (25), présentant ainsi une texture irrégulière indiquant l'emplacement général des éléments de préhension sous la mousse comprimée ; et/ou les éléments de préhension (25) étant conçus pour passer de la configuration de préhension inactive à une configuration de préhension active lors de la dissolution de la mousse compressible.

3. Implant composite (10) selon la revendication 1,
(a) la mousse compressible (30) dans la première configuration recouvrant en outre le corps (26) de chacun de la pluralité d'éléments de préhension (25) ; ou
(b) la première épaisseur de la mousse (30) étant supérieure ou égale à une longueur du corps (26) de chacun de la pluralité d'éléments de préhension (25) ; ou
(c) la mousse compressible (30) dans la première configuration ne recouvrant en outre qu'une partie du corps (26) de chacun de la pluralité d'éléments de préhension (25) ; ou
(d) la première épaisseur de la mousse (30) étant inférieure à la longueur de chacun de la pluralité d'éléments de préhension (25).

4. Implant composite (10) selon la revendication 1, l'implant composite (10) comprenant en outre un espace (40) entre la première face (21) de la maille (20) et une seconde surface de la mousse compressible (30) dans la première configuration et éventuellement la seconde configuration.

5. Implant composite (10) selon la revendication 1, la maille (20) comprenant en outre au moins une fente (29), une ouverture centrale (28), ou les deux, et la mousse compressible (30) étant positionnée le long d'au moins l'une de la fente (29), de l'ouverture (28), ou des deux.

6. Implant composite (10) selon la revendication 1, la pluralité d'éléments de préhension (25) étant dissimulés à l'intérieur de la mousse compressible (30) et dans une configuration de préhension inactive à la fois dans les première et seconde configurations de mousse ; ou
les éléments de préhension (25) étant dissimulés dans la mousse compressible (30) et dans une configuration de préhension inactive dans la première configuration de mousse et étant directement exposés dans une configuration de préhension active dans la seconde configuration de mousse.

7. Implant composite (10) selon la revendication 5, comprenant en outre une maille de rabat (35) comprenant une première face de rabat et une seconde face de rabat opposée à la première face de rabat, la maille de rabat (35) s'étendant en travers de la fente (29) et comprenant une première partie de la seconde face de rabat chevauchant une première partie de la première face de rabat (21), la pluralité d'éléments de préhension (25) s'étendant depuis la première partie de la première face de maille (21), chacun de la pluralité d'éléments de préhension (25) comprenant un corps (26) s'étendant entre une première extrémité fixée et une seconde extrémité libre.

8. Procédé de fabrication d'un implant composite (10) selon l'une quelconque des revendications 1 à 7, comprenant les étapes consistant à :
combiner un agent moussant polymérique biocompatible, un plastifiant biocompatible, et un solvant, pour former une solution,
combiner la solution avec un gaz pour former un matériau en mousse humide non coulante,
appliquer le matériau en mousse humide non coulante sur au moins une seconde extrémité libre de chacun d'une pluralité d'éléments de préhension (25) s'étendant à partir d'une première face (21) d'une maille implantable (20),
sécher la mousse humide non coulante pour former l'implant composite (10) comportant une mousse compressible (30) dans une première configuration ayant une première épaisseur, la mousse compressible (30) étant conçue pour passer de la première configuration de mousse ayant la première épaisseur à une seconde configuration de mousse ayant une seconde épaisseur inférieure à la première épaisseur,
les éléments de préhension (25) étant conçus pour passer d'une première configuration de préhension inactive recouverte par la mousse (30) à une seconde configuration de préhension active lors du retrait de la mousse pour fixer les éléments de préhension (25) et/ou l'implant (10) au tissu.

9. Procédé de fabrication d'un implant composite (10) selon la revendication 8, l'agent moussant polymère biocompatible étant choisi dans le groupe constitué de gélatines, collagènes, poloxamères, polysorbates, méthylcellulose (MC), hydroxypropylméthylcellulose (HPMC), hydroxypropylcellulose (HPC), hydroxyéthylcellulose (HEC) et leurs mélanges ; et, éventuellement,
(a) l'agent moussant polymère biocompatible étant l'hydroxypropylméthylcellulose, et éventuellement (i) l'hydroxypropylméthylcellulose comprenant un degré de substitution méthyle de 1 à 2,5 ; et/ou
(b) l'agent moussant polymère biocompatible représentant une concentration dans la plage d'environ 0,1 à environ 20 pour cent en poids de la solution aqueuse.

10. Procédé de fabrication d'un implant composite (10) selon la revendication 8, le plastifiant biocompatible étant choisi dans le groupe constitué par le glycérol, le sorbitol, le xylitol, le mannitol, le propylène glycol, le polyéthylèneglycol, et leurs mélanges ; et, éventuellement,
(a) le plastifiant polymère biocompatible étant au moins un plastifiant parmi le glycérol, le sorbitol ou les deux ; ou
(b) le plastifiant biocompatible représentant une concentration dans la plage d'environ 0,5 à environ 40 pour cent en poids de la solution aqueuse.

11. Procédé de fabrication d'un implant composite (10) selon la revendication 8, le solvant étant choisi dans le groupe constitué par l'eau de source, l'eau stérile, l'eau distillée, l'eau désionisée et leurs combinaisons.

12. Procédé de fabrication d'un implant composite (10) selon la revendication 8, la solution aqueuse comprenant en outre un additif biocompatible choisi dans le groupe constitué par l'alginate, la pectine, la carraghénine, la méthylcellulose (MC), l'hydroxypropylméthylcellulose (HPMC), l'hydroxypropylcellulose (HPC), l'hydroxyéthylcellulose (HEC), la gomme de xanthane, la gomme de guar, le dextrane, l'hyaluronane, le pullulan, la maltodextrine, les gélatines, les collagènes, l'alcool polyvinylique, le polyéthylèneglycol et leurs mélanges ; et, éventuellement,
(a) l'additif biocompatible étant l'hydroxypropylméthylcellulose avec un degré de substitution méthyle de 1 à 2,5 et une substitution molaire hydroxypropyle de 0,1 à 1 ; ou
(b) l'additif biocompatible représentant une concentration dans la plage d'environ 0,1 à environ 10 pour cent en poids de la solution aqueuse.

13. Procédé de fabrication d'un implant composite (10) selon la revendication 8, la combinaison de la solution avec un gaz pour former un matériau de mousse humide non coulante comprenant l'étape consistant à fouetter la solution pour incorporer des bulles d'air dans la solution pour former le matériau de mousse humide non coulante.

14. Procédé de fabrication d'un implant composite (10) selon la revendication 8, l'application du matériau en mousse humide non coulante à au moins une seconde extrémité libre de chaque élément d'une pluralité d'éléments de préhension (25) s'étendant depuis une première face d'une maille implantable (20) comprenant l'étape consistant à couler le matériau en mousse humide non coulante sur un support inerte jusqu'à une épaisseur suffisante pour recouvrir au moins l'extrémité libre de chaque pluralité d'éléments de préhension (25) et le placement de la maille implantable (20) avec les éléments de préhension (25) orientés vers le matériau humide non coulant sur une première surface supérieure du matériau humide non coulant.

15. Procédé de fabrication d'un implant composite (10) selon la revendication 8, le séchage du matériau en mousse humide non coulante comprenant l'étape consistant à chauffer le matériau en mousse humide non coulante dans un four à environ 50 °C.
